# EUROPEAN PATENT SPECIFICATION

(11) **EP 2 486 905 B1**
(45) Date of publication and mention of the grant of the patent: **19.04.2017**
(21) Application number: 10822043.5
(22) Date of filing: 06.10.2010
(51) Int. Cl.: A61F 13/496, A61F 13/15, A61F 13/494, A61F 13/514

(54) **PULL-UP TYPE ABSORPTIVE ARTICLE**
HOCHZIEHBARER SAUGFÄHIGER ARTIKEL
ARTICLE ABSORBANT DE TYPE CULOTTE

(30) Priority: 08.10.2009 JP 2009234638
(43) Date of publication of application: 15.08.2012
(73) Proprietor: Kao Corporation, Chuo-Ku Tokyo 103-8210 (JP)
(72) Inventor: SASAKI, Jun, Haga-gun Tochigi 321-3497 (JP); ONDA, Aiko, Haga-gun Tochigi 321-3497 (JP); SONO, Tokihito, Haga-gun Tochigi 321-3497 (JP)
(74) Representative: Vossius & Partner Patentanwälte Rechtsanwälte mbB
(86) International application number: PCT/JP2010/067544
(87) International publication number: WO 2011/043373

(56) References cited:
- WO-A1-2008/108270
- JP-A- 8 038 546
- JP-A- 2004 329 590
- JP-A- 2007 509 725
- JP-A- 2008 194 161
- JP-A- 2008 272 426
- JP-A- 2009 106 666
- JP-A- 2009 160 129
- JP-A- 2009 240 639
- JP-A- 2009 240 640
- JP-A- 2009 511 159
- US-A1- 2004 122 412
- US-A1- 2004 243 086

## Description

### Technical Field

The present invention relates to a pull-on (pants type) absorbent article, such as a disposable diaper.

### Background Art

A pull-on absorbent article is known, which includes an hourglass-shaped outer cover having a front portion to be worn about the wearer's front, the crotch portion to be worn about the wearer's crotch, and a rear portion to be worn about the wearer's rear and an absorbent assembly fixed to the inner side of the outer cover, with both the lateral side edges of the outer cover in the front portion and those in the rear portion being joined together to form a waist opening and a pair of leg openings.

Continuous production of such pull-on absorbent articles generally includes the steps of making through-holes or cutouts in a continuous length of outer cover (trimming step) to form leg openings and removing the unnecessary parts, i.e., trimmings.

Conventional pull-on absorbent articles include those in which the outer cover is separated into a front side panel to be worn about the front side of a wearer and a rear side panel to be worn about the rear side of the wearer, and the absorbent assembly is fixed to bridge the front side panel and the rear side panel, with both lateral side edges of the front side panel and those of the rear side panel being joined together (see patent literatures 1 to 3 below).

Patent literature 4 below discloses an example of that type of pull-on absorbent articles, which has an annular elastic belt composed of a front belt portion and a back belt portion and an absorbent body, the back belt portion (i.e., rear side panel) having a larger length in the longitudinal direction than the front belt portion (i.e., front side panel) in the longitudinal direction.

### Citation List

### Patent Literature

Patent literature 1: EP2186494A1
Patent literature 2: JP 2008-194161A
Patent literature 3: WO2005/051264
Patent literature 4: WO2006/017718
Patent literature 5: WO2004/108039

Also US2004/122412 A1 discloses relevant prior art.

### Summary of Invention

### Technical Problem

Although pull-on absorbent articles of the type wherein the outer cover is separated into a front panel to be worn about the front of a wearer and a rear panel to be worn about the back of the wearer offer the advantage of removing the need to trim a continuous length of outer cover or reducing the size of unnecessary parts to be trimmed off, they frequently look like out-of-date loincloth while worn. This appearance often evokes a feeling of insecurity about leakage, which is a matter of concern in absorbent articles, and does not suit many consumers' preference. The pull-on absorbent articles of patent literatures 1 to 3 have this problem of appearance.

In particular, the pull-on absorbent article of patent literature 3 is designed such that the angle between the lower edge of the rear panel and the side edge of the absorbent body (absorbent assembly) is greater in a contracted state than in an uncontracted state. As a result, the rear panel tends to tilt its lower edge upward toward both lateral sides of the wearer while worn to uncover part of the wearer's buttocks.

The pull-on garment of patent literature 4 provides improved coverage on wearer's buttocks because of the longer length of the back belt portion (rear panel) than the front belt portion (front panel). However, because for one thing the absorbent body becomes narrower on being pressed from both sides by the wearer's thighs and, for another thing, the extent of lengthening the back belt portion has a limit, part of the wearer's buttocks is liable to be exposed below the lower edge of the back belt portion and outward from both side edges of the absorbent body. Thus, the pull-on garment has poor aesthetics and looks like a leaky diaper when seen from the back and sides of the wearer.

The pant-like absorbent article of patent literature 5 includes a front panel and a back panel each having a hexagonal shape with the sides closest to the crotch portion tilted largely with respect to the transverse direction of the article. This design is unfavorable in terms of simplification of manufacturing steps and reduction of unnecessary parts to be trimmed off the respective continuous sheet materials.

The invention relates to a pull-on absorbent article which is of the type wherein the outer cover is separated into a front panel adapted to be worn about the front of a wearer and a rear panel adapted to be worn about the rear of the wearer and yet provides an improved appearance from the back and side of a wearer.

The invention also relates to a pull-on absorbent article which is of the type wherein the outer cover is separated into a front panel adapted to be worn about the front of a wearer and a rear panel adapted to be worn about the rear of the wearer and yet provides good coverage over the wearer's buttocks.

### Solution to Problem

Described is a pull-on absorbent article including a front panel adapted to be worn about the front of a wearer, a rear panel adapted to be worn about the rear of a wearer, and an absorbent assembly fixed to the front and the rear panel so as to bridge them. The article has a pair of side seals formed by joining the front panel and the rear panel along their lateral side edges. The rear panel has an oblong rectangular shape, is longer than the side seals in the longitudinal direction of the absorbent article and includes a rear extension portion extending downward from the side seals. The absorbent assembly includes an oblong absorbent member and a side sheet portion that is located laterally outward from each lateral side edge of the absorbent member. Each side sheet portion has an elastic member to form a side gather in at least the crotch portion of the absorbent article. Each side sheet portion has a non-bonded region not bonded to the rear extension portion in the portion thereof overlapping the rear panel and nearer the crotch portion. The elastic member extends in the non-bonded region so that the side sheet portion is elasticized in the non-bonded region, too.

Described is a pull-on absorbent article including a front panel adapted to be worn about applied to the front of a wearer, a rear panel adapted to be worn about the rear of a wearer, and an absorbent assembly fixed to the front and the rear panel so as to bridge them. The article has a pair of side seals formed by joining the front panel and the rear panel along their lateral side edges. The rear panel has an oblong rectangular shape and includes a rear extension portion extending downward from the side seals. The absorbent assembly includes an oblong absorbent member and a side sheet portion located laterally outward from each lateral side edge of the absorbent member. Each side sheet portion has an elasticized region having an elastic member fixed thereto in a stretched state. The elasticized region extends in the crotch portion of the article to be worn about the crotch of a wearer and on the rear extension portion. The elasticized region is bonded over at least a part of its width to at least the rear extension portion of the rear panel to form a rear longitudinally elasticized region. In a relaxed state of the absorbent article with the rear longitudinally elasticized region contracted, the lower edge of the rear panel has a downward concave near the rear longitudinally elasticized region.

Described is a pull-on absorbent article including a front panel adapted to be worn about applied to the front of a wearer, a rear panel adapted to be worn about the rear of a wearer, and an absorbent assembly fixed to the front and the rear panel so as to bridge them. The article has a pair of side seals formed by joining the front panel and the rear panel along their lateral side edges. The rear panel has an oblong rectangular shape and includes a rear extension portion extending downward from the side seals. The absorbent assembly includes an oblong absorbent member, a side sheet portion laterally outward from each lateral side edge of the absorbent member, and a standing gather-forming portion forming a standing gather laterally inwardly from each side sheet portion. Each side sheet portion has an elasticized region with an elastic member fixed thereto in a stretched state. The elasticized region extends in the crotch portion of the article to be worn about the crotch of a wearer and on the rear extension portion. The elasticized region is not bonded, over the part of its width where the elastic member is fixed, to the rear extension portion. The standing gather-forming portion is configured to form the standing gather in the crotch portion and on the rear extension portion. The rear panel has a rear longitudinally elasticized region that is extensible and contractible in the longitudinal direction of the absorbent assembly in at least the rear extension portion thereof near each side edge of the absorbent member. In a relaxed state of the absorbent article with the rear longitudinally elasticized region contracted, the lower edge of the rear panel has a downward concave near the rear longitudinally elasticized region.

### Brief Description of Drawings

[Fig. 1] FIG. 1(a), FIG. 1(b), and FIG. 1(c) each illustrate a disposable pull-on diaper according to a first embodiment of the invention (an embodiment of the first aspect of the invention) while in use (while worn), of which FIG 1(a) is a view from the front of a wearer, FIX 1(b) is a side view, and FIG. 1(c) is a back view.
[Fig. 2] FIG. 2 is a plan of the disposable pull-on diaper shown in Figs. 1(a) to 1(c) in its flat-out, uncontracted state with a part cut away. As used herein, the term "flat-out, uncontracted state" means a state in which a pull-on absorbent article is opened by tearing the side seals apart and with every elastic member straightened up to its design dimension (the dimension of an article in a flat-out configuration with any influences of elastic members eliminated).
[Fig. 3] FIG 3 is an enlarged cross-section taken along line III-III of Fig. 2.
[Fig. 4] FIG. 4 is an enlarged cross-section taken along line IV-IV of Fig. 2.
[Fig. 5] FIG 5 is an enlarged cross-section taken along line V-V of Fig. 2.
[Fig. 6] FIG. 6 is an enlarged cross-section taken along line VI-VI of Fig. 2.
[Fig. 7] FIG. 7 shows an absorbent assembly viewed from the inside of a disposable pull-on diaper according to a second embodiment of the invention in its flat-out, uncontracted state.
[Fig. 8] FIG. 8(a), FIG. 8(b), and FIG. 8(c) show the disposable pull-on diaper according to the second embodiment of the invention, of which Fig. 8(a) is a schematic cross-section taken along line A1-A1 in Fig. 7 (in a relaxed state), FIG. 8(b) is a schematic cross-section taken along line B1-B1 in Fig. 7, and FIG. 8(c) is a schematic cross-section along line C1-C1 in Fig. 7.
[Fig. 9] FIG. 9 shows an absorbent assembly viewed from the inside of a disposable pull-on diaper according to a third embodiment of the invention in its flat-out, uncontracted state.
[Fig. 10] FIG. 10(a), FIG. 10(b), and FIG. 10(c) show the disposable pull-on diaper according to the third embodiment of the invention, of which Fig. 10(a) is a schematic cross-section taken along line A2-A2 in Fig. 9 (in a relaxed state), FIG. 10(b) is a schematic cross-section taken along line B2-B2 in Fig. 9, and FIG. 10(c) is a schematic cross-section taken along line C2-C2 in Fig. 9.
(Fig. 11] FIG. 11(a) and FIG. 11(b) illustrate a disposable pull-on diaper according to a fourth embodiment of the invention (an embodiment of a second aspect of the invention) in its relaxed state, of which FIG. 11(a) is a view from the side of the front panel, and FIG 11(b) is a view from the side of the rear panel.
[Fig. 12] FIG. 12 is a plan of the disposable pull-on diaper of Figs. 11(a) and 11(b) in its flat-out, uncontracted state.
[Fig. 13] FIG. 13 is an enlarged cross-section taken along line III-III in Fig. 12.
[Fig. 14] Fig. 14 is an enlarged cross-section taken along line IV-IV in Fig. 12.
[Fig. 15] FIG. 15 is an enlarged cross-section taken along line V-V in Fig. 12.
[Fig. 16] FIG. 16 is a perspective view diagonally behind of the disposable pull-on diaper of Figs. 11 (a) and 11 (b) while worn.
[Fig. 17] FIG. 17(a), FIG. 17(b), and FIG. 17(c) illustrate a disposable pull-on diaper according to a fifth embodiment of the invention, of which FIG. 17(a) is a view of the absorbent assembly from the inside of the diaper in its flat-out, uncontracted state,
FIG. 17(b) is a schematic cross-section taken along line Xa-Xa in Fig. 17(a) (in a relaxed state), and FIG. 17(c) is a schematic cross-section taken along line Xb-Xb in Fig. 17(a).
[Fig. 18] FIG. 18 is a schematic plan of a disposable pull-on diaper according to a sixth embodiment of the invention in its flat-out, uncontracted state.
[Fig. 19] FIG. 19(a) and FIG. 19(b) are cross-sections of the disposable pull-on diaper of Fig. 18, of which FIG. 19(a) is an enlarged cross-section taken along line Ya-Ya in Fig. 18, and FIG. 19(b) is an enlarged cross-section taken along line Yb-Yb in Fig. 18.
[Fig. 20] FIG. 20 is a schematic plan of a disposable pull-on diaper according to a seventh embodiment of the invention (an embodiment of a third aspect of the invention) in its flat-out, uncontracted state.
[Fig. 21] FIG. 21(a) and FIG. 21(b) are cross-sections of the disposable pull-on diaper of Fig. 20, of which FIG. 21(a) is an enlarged cross-section taken along line Za-Za in Fig. 20, and FIG. 21(b) is an enlarged cross-section taken along line Zb-Zb in Fig. 20.
[Fig. 22] FIG. 22 is a view of a conventional disposable pull-on diaper while worn (equivalent to Fig. 16).

### Description of Embodiments

The invention will be described based on its preferred embodiments with reference to the accompanying drawings.

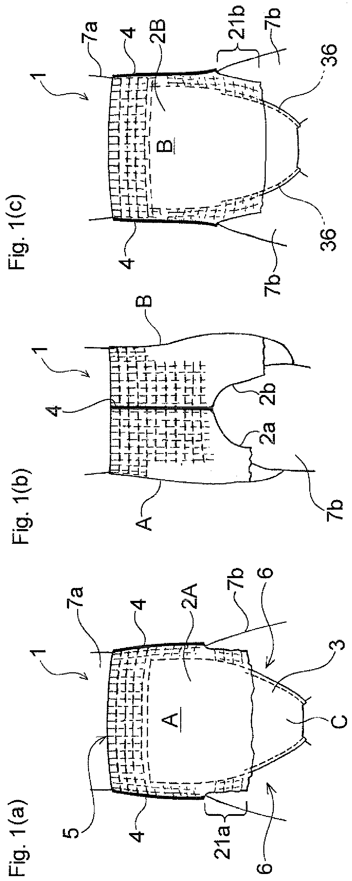

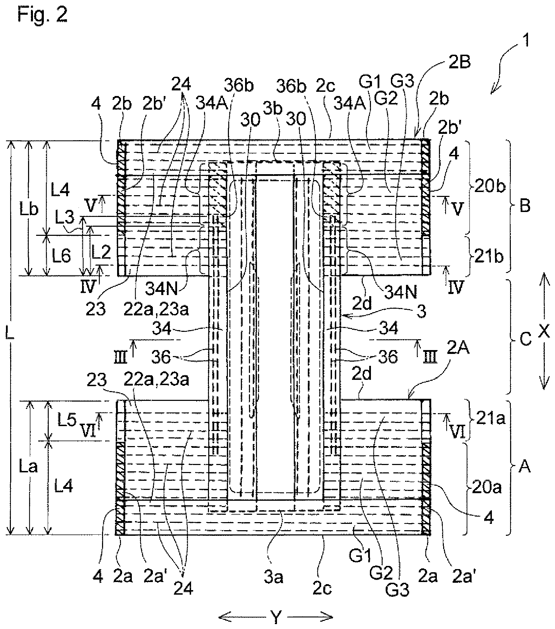

As shown in Figs. 1 and 2, a disposable pull-on diaper 1 according to a first embodiment of the invention (hereinafter simply referred to as "diaper 1") includes a front panel 2A adapted to be worn about the wearer's front and having opposite lateral side edges 2a and 2a, a rear panel 2B adapted to be worn about the wearer's rear and having opposite lateral side edges 2b and 2b, and an absorbent assembly 3 fixed to the front panel 2A and the rear panel 2B so as to bridge them. The lateral side edges 2a and 2a of the front panel 2A and the lateral side edges 2b and 2b of the rear panel 2B are joined together, respectively, to form a pair of opposite side seals 4 and 4.

The front panel 2A has a length La greater than the length L4 of the side seals 4 in the longitudinal direction (direction X) of the diaper (i.e., absorbent article). That is, the front panel 2A has a front extension portion 21a extending downward (while worn) from the side seals 4. The rear panel 2B has a length Lb greater than the length L4 of the side seals 4 in the longitudinal direction (direction X) of the diaper (i.e., absorbent article). That is, the rear panel 2B has a rear extension portion 21b extending downward (while worn) from the side seals 4. The portion of the front panel 2A that is located between the opposite side seals 4 and 4 (the portion other than the front extension portion 21 a) is called the front panel main portion 20a. The portion of the rear panel 2B that is located between the opposite side seals 4 and 4 (the portion other than the front extension portion 21b) is called the rear panel main portion 20b.

In describing an absorbent article, the tem "upward" and the term "downward" mean toward the side of the waist opening 5 and toward the side of the leg openings 6 and 6, respectively, while the absorbent article is worn.

The diaper 1 will be described in detail. As shown in Figs. 1 and 2, the diaper 1 has a front portion A worn about the wearer's front, a rear portion B worn about the wearer's rear, and the crotch portion C located between the front portion A and the rear portion B and worn about the wearer's crotch while worn. The diaper 1 (absorbent article) has a longitudinal direction (direction X in Fig. 2) extending from the front portion A through the crotch portion C to the rear portion B or vice versa and a lateral direction (direction Y in Fig. 2) perpendicular to the longitudinal direction.

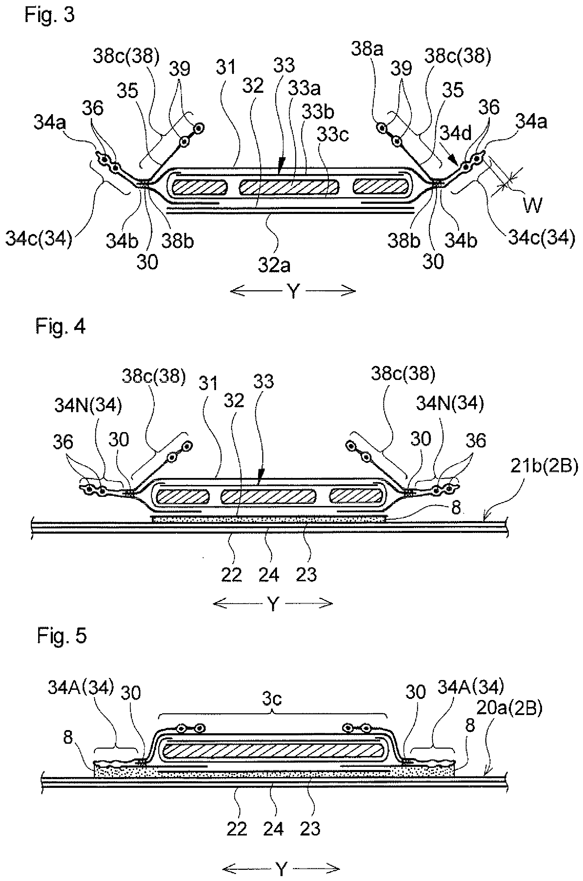

The absorbent assembly 3 of the diaper 1 has a rectangular shape longer in direction X as shown in Fig. 2. As shown in Fig. 3, the absorbent assembly 3 includes a liquid permeable topsheet 31, a liquid impermeable or water repellent backsheet 32, and a liquid retentive absorbent member 33 interposed between the two sheets 31 and 32. The absorbent member 33 is composed of an absorbent core 33a formed of an aggregate of fibers (e.g., pulp fiber), which may be nonwoven fabric, with or without a superabsorbent polymer incorporated therein, and core wrap sheets 33b and 33c wrapping the absorbent core 33a. The absorbent member 33 is also rectangular, longer in direction X.

As shown in Fig. 3, the absorbent assembly 3 has along both lateral sides thereof a pair of side sheet portions 34 and 34 located laterally outwardly from the lateral side edges of the absorbent member. Each side sheet portion forms a side gather 34c in at least the crotch portion C (i.e., the part of the side sheet portion that extends in the longitudinal direction (direction x) of the absorbent assembly 3 between the front panel 2A and the rear panel 2B).

As shown in Fig. 2, each side sheet portion 34 extends over the whole length of the absorbent assembly 3. As shown in Fig. 3, each side sheet portion 34 includes a liquid resistant or water repellent sheet 35 and elastic members 36 and 36 fixed to the sheet 35. Each elastic member 36 is fixed in its stretched state to at least the crotch portion C to form a side gather 34c in at least the crotch portion C. The side gather 34c is a result of the contraction of the elastic members 36 provided in the side sheet portion 34. On contraction of the elastic members 36, the sheet 35 forming the side sheet portion 34 gathers or is deformed into a wavy cross-section to form the side gather 34c.

It suffices that the side gather 34c be formed while the diaper is in a relaxed state and/or during wear. While worn, the part of each side sheet portion that exists in the crotch portion C rises toward the wearer's skin starting from the distal edge of a linear bond 30 (hereinafter described) as a base 34b of rising.

The elastic member 36 is disposed so as to form a gather extensible generally in the longitudinal direction of the absorbent assembly 3. While the elastic member 36 is preferably disposed in parallel with direction X as shown in Fig. 2, it may be somewhat angled with respect to direction X or curved laterally inwardly or outwardly. While the elastic member 36 is fixed to the sheet 35 preferably continuously in the longitudinal direction of the absorbent assembly 3, the fixing may be discontinuous as long as a side gather is formed.

The absorbent assembly 3 used in the diaper 1 of the present embodiment has along its sides a pair of standing gather-forming portions 38 extending in the longitudinal direction of the absorbent assembly 3.

Each standing gather-forming portion 38 includes a sheet 35 and standing gather-forming elastic members 39 fixed to the sheet 35. The standing gather-forming portion 38 forms a standing gather 38c in at least the crotch portion C. In the diaper 1 of the present embodiment, the sheet 35 is a double layer laminate sheet formed by folding a single sheet onto itself along a fold line corresponding to the free edge 38a of the standing gather 38c. The elastic members 39 are fixed with an adhesive between the two layers. The laminate sheet is also folded onto itself along a fold line corresponding to the free edge 34a of the side gather 34c, and the elastic members 36 are fixed with an adhesive between the facing two layers of the laminate sheet. The two layers of the laminate sheet are bonded together with the side edge of the topsheet 31 inserted therebetween along a linear bond 30 near, more specifically along a position slightly outward from, each side edge of the absorbent member 33.

As shown in Fig. 2, the linear bond 30 extends along each side edge of the rectangular absorbent member 33 over the whole length of the absorbent assembly 3 as well as the absorbent member 33 in direction X. The linear bond 30 is preferably a continuous straight line but may be a dotted line. The linear bond 30 may be formed by various known methods, such as heat sealing, ultrasonic sealing, high frequency sealing, or application of an adhesive.

The standing gather 38c rises toward the wearer's skin while the diaper 1 is worn to block a laterally outward flow of a fluid. The topsheet 31, backsheet 32, absorbent core 33a, and the core wrap sheets 33b and 33c may be of any materials conventionally used in this type of absorbent articles. To provide an improved feel to the touch, an outer cover 32a made of nonwoven fabric or the like may be provided on the side of the backsheet 32 of the absorbent assembly 3.

In a flat-out, uncontracted state of the diaper 1 (see Fig. 2), the front panel 2A has a rectangular shape longer in the lateral direction and has opposite lateral side edges 2a and 2a extending in the diaper longitudinal direction (direction X) and opposite longitudinal end edges 2c and 2d extending in the diaper lateral direction (direction Y). Similarly, the rear panel 2B has a rectangular shape longer in the lateral direction, opposite lateral side edges 2b and 2b extending in the diaper longitudinal direction (direction X), and opposite longitudinal end edges 2c and 2d extending in the diaper lateral direction (direction Y). As depicted in Fig. 2, the length of each of the front panel 2A and the rear panel 2B in the diaper longitudinal direction or direction X is uniform in the diaper lateral direction (direction Y).

The front panel 2A and the rear panel 2B are joined together along a part 2a' of each side edge 2a of the former and a part 2b' of each side edge 2b of the latter, whereby the diaper 1 has a pair of side seals 4 and 4, a waist opening 5, and a pair of leg openings 6 and 6. Joining the front and the rear panel is achieved by, for example, heat sealing, high frequency sealing, ultrasonic sealing, or adhesive application. In Figs. 1(a) through 1(c) are shown the waist opening 5 and the leg openings 6 and 6 through which the torso 7a and legs 7b and 7b of a child extend, respectively.

The front and the rear panel 2A and 2B of the diaper 1 each include an outer sheet 22 that defines the exterior of the diaper, an inner sheet 23 laid on the inner side of the outer sheet 22, and a plurality of elastic members 24 of thread form spacedly arranged to extend in direction X between the two sheets 22 and 23 as shown in Figs. 4 and 5. The front and the rear panel 2A and 2B each have an elasticized waist region G1, an elasticized below-waist region G2, and an elasticized extension region G3 as shown in Fig. 2.

In embodiments where the front and the rear panel 2A and 2B are elasticized by the elastic members 24 disposed between the two sheets 22 and 23 in their stretched state, it is preferred for the opposite side edges 2a and 2a and for the opposite side edges 2b and 2b to be parallel with each other with the elastic members 24 in their stretched state as shown in Fig. 2. Each of the front and the rear panel 2A and 2B is preferably rectangular longer in the diaper lateral direction with the elastic members 24 in their stretched state.

The elasticized waist region G1 is outward from the longitudinal end 3a or 3b of the absorbent assembly 3 in the diaper longitudinal direction (direction X) in each of the front and the rear panels 2A and 2B. The elasticized below-waist region G2 is between the elasticized waist region G1 and the extension portion 21a or 21b along direction X in each of the front and the rear panel 2A and 2B. The elasticized extension region G3 is fonned in the front extension portion 21a and in the rear extension portion 21b.

It is preferred that each of the elasticized waist region G1, elasticized below-waist region G2, and elasticized extension region G3 be elasticized in at least a region located laterally outwardly from each lateral (direction Y) side edge of the absorbent member 33.

While the embodiment shown in Figs. 1 through 5 has the elastic members 24 extending over the whole width of the front and the rear panel 2A and 2B across the absorbent member 33 to form the waist elasticized region G1, elasticized below-waist region G2, and elasticized extension region G3, it is preferred that the elastic members 24 forming the elasticized below-waist region G2 and/or the elasticized extension region G3 be deelasticized by heating, cutting, or like means in an area overlapping a laterally middle portion of the absorbent member 33 so as not to elasticize that part of the front and rear panel 2A and 2B.

The two sheets 22 and 23 making up the front and rear panels 2A and 2B may be bonded to each other with an adhesive over substantially the entire area thereof or, as with the case of the elasticized laminate sheet described in JP 2005-80859A, be bonded at a large number of bonds discretely arranged therebetween in a pattern that does not overlap the elastic members 24.

The absorbent assembly 3 is fixed at one longitudinal end portion thereof (the portion overlapping the front panel 2A) to the lateral (direction Y) middle portion of the front panel 2A with an adhesive 8, and is fixed at the opposite end portion (the portion overlapping the rear panel 2B) to the lateral (direction Y) middle portion of the rear panel 2B with an adhesive 8.

In the diaper 1 of the present embodiment, the part of each side sheet portion 34 has a bonded region 34A bonded to the rear panel 2B in a portion thereof overlapping the rear panel 2B and nearer the longitudinal end 3b of the absorbent assembly 3 as shown in Figs. 2 and 5. Each side sheet portion 34 is fixed at the bonded region 34A in its laterally (direction Y) outwardly lying position to the rear panel 2B via an adhesive 8.

On the other hand, the part of each side sheet portion 34 that is located on the rear panel 2B and nearer the crotch portion C is not bonded to the rear extension portion 21B to provide a non-bonded region 34N as shown in Figs. 2 and 4. It is preferred that the non-bonded region 34N not be bonded to the rear panel 2B in at least a part thereof laterally outward from the position of the elastic members 36. More preferably, the part of the non-bonded region 34N that is laterally outward from the linear bond 30 is not bonded to the rear panel 2B. The non-bonded region 34N preferably has a width (a dimension in direction Y in the state shown in Fig. 2) of 5 to 40 mm, more preferably 10 to 35 mm, even more preferably 15 to 35 mm.

The non-bonded region 34 has a length L2 from the lower edge 2d of the rear panel 2B in direction X (see Fig. 2). The ratio of the length L2 of the non-bonded region 34N to the length L6 of the rear extension portion 21B (hereinafter "extension length L6"), L2/L6, is preferably 0.5 to 1.5, more preferably 0.7 to 1.2. For use by children, the length L2 of the non-bonded region 34N is preferably 25 to 75 mm, more preferably 35 to 60 mm. For use by adults, the length L2 of the non-bonded region 34N is preferably 35 to 115 mm, more preferably 50 to 90 mm.

The absorbent assembly 3 preferably has its lateral middle portion 3c where the absorbent member 33 is disposed bonded to the rear panel main portion 20b and the rear extension portion 21b with, for example, an adhesive 8. By this bonding, the rear panel 2B is prevented from separating from the absorbent assembly 3, which raises the perfection level of aesthetic appearance. Furthermore, the adhesive bonding improves stiffness, which is effective to maintain the width of the absorbent core and thereby to prevent leakage.

In the present embodiment, the elastic members 36 forming each side gather 34c extend also through the non-bonded region 34N so that the side sheet portion 34 is elasticized in the non-bonded region 34N as well.

The elastic members 36 disposed in each side sheet portion 34 are fixed to the sheet 35 in their stretched state to form a side gather 34c together with the sheet 35 in at least the crotch portion C. The elastic members 36 further extend to overlap the rear panel 2B and are fixed to the sheet 35 in their stretched state in the non-bonded region 34N. Therefore, the non-bonded region 34N is also elasticized. The elastic members 36 that form the side gather 34c are disposed in the side sheet portion 34 along the longitudinal direction of the side sheet portion and contract to gather the sheet 35 to form a gather in at least the crotch portion C. In the case where any elastic member 36 has one or two ends thereof free without being secured to the sheet 35, such a free end is not included in the elastic member 36 forming the side gather 34c.

The part of the elastic member 36 that overlaps the rear panel 2B has a length L3 (a distance from the lower edge 2d of the rear panel 2B to the end 36b of the elastic member 36 on the rear panel 2B in direction X, see Fig. 2). The ratio of the length L3 to the length L2 of the non-bonded region 34N, L3/L2, is preferably 0.5 to 2.0, more preferably 0.7 to 1.5. For use by children, the length L3 of the elastic member 36 is preferably 25 to 100 mm, more preferably 35 to 75 mm. For use by adults, the length L3 is preferably 30 to 150 mm, more preferably 50 to 120 mm.

Since the diaper 1 of the present embodiment has the rear extension portion 21b in the rear panel 2B, the position of the lower edge 2d of the rear panel 2B on the wearer's body is lower than with a conventional diaper in which both side edges of the front panel 2A and both side edges of the rear panel 2B are joined over the total length in the diaper longitudinal direction. As a result, it is less likely that part of the wearer's buttocks bulges from under the lower edge 2d of the rear panel 2B or from the side of the absorbent assembly 3, which is one of the problems to be settled.

Moreover, the side sheet portions 34 along the sides of the absorbent assembly 3 help the absorbent assembly to maintain a sufficient width near the lower edge 2d of the rear panel 2B. This brings about improvement on the problem that the absorbent assembly is liable to be squeezed and narrowed between the wearer's thighs.

Additionally, each side sheet portion 34 has the non-bonded region 34N that is located on the rear panel 2B and nearer the crotch portion C and is not bonded to the rear extension portion 21B. Therefore, even though the non-bonded region 34N contracts, the contracting force of the non-bonded region 34N does not cause the rear extension portion 21B to contract in the diaper longitudinal direction. Extensibility and contractibility of the side sheet portions is important to eliminate any gap with the thigh circumference to prevent leakage of bodily discharges, and it is necessary to dispose the elastic member as long as possible in the longitudinal direction of the side sheet portion 34. On the other hand, the position of the lower edge 2d of the rear extension portion 21b is important to prevent part of the wearer's buttocks from bulging (being exposed excessively). Since the rear extension portion 21b, being free from the contracting force of the non-bonded region 34N, does not contract in the longitudinal direction, the position of the lower edge 2d of the rear panel 2B is kept low. Thus, the structure of the diaper 1 achieves effective and reliable improvement on the prevention of the wearer's buttocks bulging while maintaining absorbing performance (elimination of the gap with the thigh circumference and prevention of leakage).

Since each side sheet portion 34 is fixed in its laterally outwardly lying position to the rear panel main portion 20b, the absorbent assembly 3 has a sufficient width near the lower edge 2d of the rear panel 2B to provide further improved coverage on the wearer's buttocks.

In the diaper 1 of the present embodiment, a plurality of elastic members 36 are provided in each side sheet portion 34 between the front panel 2A and the rear panel 2B, and all these elastic members 36 are disposed closer to the distal free edge 34a than the lateral center 34d of the side gather 34c. The lateral center 34d of the side gather 34c is the position at which the distance from the proximal fixed end 34b to the distal free edge 34a of the side gather 34c is divided into equal halves in a flat-out, uncontracted state of the diaper 1.

The distance from the proximal fixed end 34b to the distal free edge 34a of the side gather 34c (in direction Y in Fig. 2) is preferably 10 to 40 mm, more preferably 20 to 40 mm.

The side sheet portion 34 in the crotch portion C (i.e., the side gather 34c) rises toward the wearer's skin starting from the distal end of the linear bond 30, i.e., the fixed end 34b. Because the elastic members 36 are disposed near along the free edge 34a, that is, there are no elastic members near the fixed end 34b, the side gather 34c exhibits improved rising properties to come into good contact with the wearer's skin. The side gather that is free standing into contact with the skin prevents bodily fluids that run off the edge of the standing gather 38c from further running out of the diaper. The good contact with the wearer's skin eliminates a gap between the diaper and the leg circumference in the crotch portion, which has the advantage that mothers' anxiety about leakage is removed to reassure them. The number of the elastic members 36 is preferably at least two per side sheet portion 34 so that the side gather 34c may come into planar contact with the wearer's skin (groin) to further enhance the above described effects.

In the diaper 1 of the present embodiment, the elastic members 36 forming the side gather 34c are not provided just along the free edge 34a of the side gather 34c. In other words, the elastic member 36 closest to the free edge 34a is disposed at a prescribed distance W from the free edge 34a as shown in Fig. 3. By this arrangement, the side gather contacts the skin on its region slightly inward from the free edge 34a with the free edge 34a ruffling to prevent a gap from creating around the leg circumference even if the diaper slides down after, for example, urination. Furthermore, the very edge of the side gather 34c is not hard because of the absence of the elastic member, thereby preventing red marks on the wearer's skin and discomfort while the diaper 1 is worn.

The distance W is preferably 2 to 20 mm, more preferably 3 to 15 mm. In the case when the sheet is folded back at a position where an elastic member is disposed, and the folded edge forms the free edge 34a, the elastic member is deemed to be disposed just along the free edge 34a.

Since the diaper 1 of the present embodiment has a front extension portion 21a in the front panel 2A, it has a good appearance from the side of the front portion A without looking like loincloth.

Because the elasticized extension region G3 formed in each of the front extension portion 21a and the rear extension portion 21b contracts to a moderate degree by the elastic members 24 disposed therein, when the diaper 1 is fitted to a wearer, the side edge 2a of the front extension portion 21a of the front panel 2A and the side edge 2b of the rear extension portion 21b of the rear panel 2B on each side of the diaper 1 separate from each other toward the front and the rear in the shape of an inverted letter Y as shown in Fig. 1(b). Therefore, the diaper 1 has a good appearance from the side and looks like providing good protection against leakage.

Because the front extension portion 21a of the front panel 2A forms the elasticized extension region G3 that extends and contracts in the diaper lateral direction, the front extension portion 21 a comes into planar contact to the skin to provide coverage on the groins. After urination, the increased weight is supported by the front extension portion 21a, the front panel main portion 20a above the front extension portion 21a, and the rear panel main portion 20b. As a result, the pressure is diffused to prevent leaving red marks.

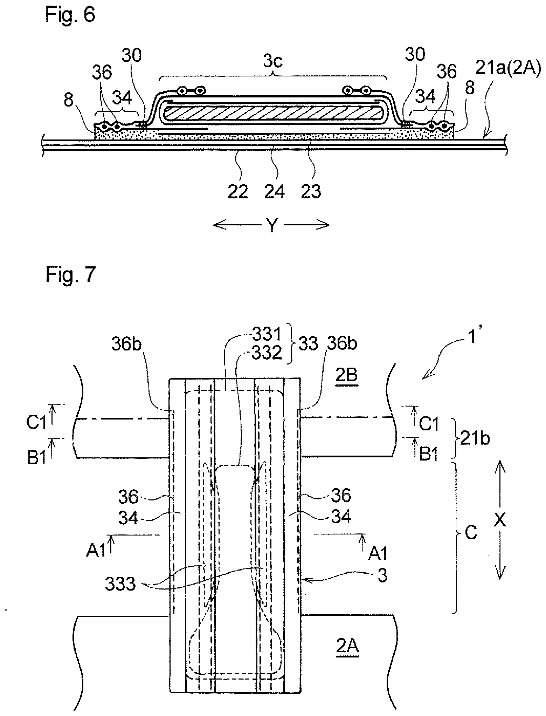

In the diaper 1 of the present embodiment, each side sheet portion 34 is fixed in its laterally (direction Y) outwardly lying position to the front extension portion 21a of the front panel 2A as shown in Figs. 2 and 6. More specifically, each side sheet portion 34 is fixed in its laterally (direction Y) outwardly lying position to the front sheet panel 2A over its entire area overlapping the front panel 2A inclusive of the portion overlapping the front panel main portion 20a.

Each side sheet portion 34 is also elasticized in its part overlapping the front panel 2A. Specifically, the side gather-forming elastic members 36 are also disposed in the part of the side sheet portion 34 that is fixed to the front panel 2A to elasticize that part.

Thus, the elastic members 24 of the front panel 2A and the elastic members 36 of the side sheet portions 34 and 34 cooperate with each other to provide a better fit to the wearer's body.

In embodiments in which the front panel 2A has the front extension portion 21a, each of the side sheet portions 34 is preferably fixed to the front extension portion 21 a in the above described fashion. In embodiments in which the front panel 2A does not have the front extension portion 21 a, each side sheet portion 34 is preferably fixed to the lower part of the front panel 2A (the part nearer the lower edge 2d of the front panel 2A) in the above described fashion.

In the diaper 1 of the present embodiment and a diaper 1A according to the fourth embodiment hereinafter described, the outer sheet 22 and the inner sheet 23 are folded along the waist opening edge, i.e., the diaper end edges 2d over the inner sheet 23 to form folded-over panels 22a and 23a, respectively. The folded-over panels 22a and 23a are bonded to the facing inner sheet 23 at the side seals 4 and to the topsheet 31 of the facing absorbent assembly 3 with an adhesive.

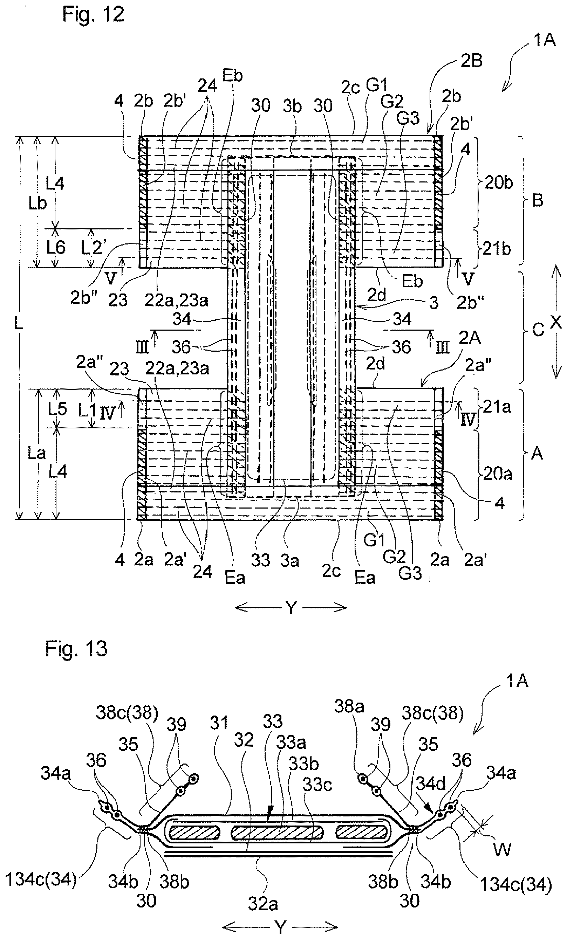

It is preferred that the front extension portion 21a have an extension length L5 (the length in the diaper longitudinal direction, see Figs. 2 and 12) of from 5% to 60%, more preferably 5% to 50%, even more preferably 10% to 40%, most preferably 20% to 40%, of the length La of the front panel 2A (the length in the diaper longitudinal direction, see Figs. 2 and 12). It is preferred that the extension portion 21b have an extension length L6 (the length in the diaper longitudinal direction, see Figs. 1 and 12) of from 5% to 60%, more preferably 10% to 50%, even more preferably 20% to 40%, of the length Lb of the rear panel 2B in the same direction. For use by children, the extension lengths L5 and L6 of the front and rear extension portion 21a and 21b, respectively, are preferably 10 to 100 mm, more preferably 10 to 70 mm, even more preferably 20 to 70 mm, most preferably 20 to 60 mm. For use by adults, the extension lengths L5 and L6 of the front and rear extension portion 2 lea and 21b, respectively, are preferably 10 to 150 mm, more preferably 15 to 110 mm, even more preferably 20 to 100 mm, most preferably 30 to 90 mm.

The length La of the front panel 2A is preferably 10% to 40%, more preferably 15% to 35%, of the total diaper length L. The length Lb of the rear panel 2b is preferably 20% to 40%, more preferably 25% to 35%, of the total diaper length L.

The extension lengths L5 and L6 of the front extension portion 21a and the rear extension portion 21b, respectively, are preferably in the relation L5<L6 to help visually distinguish the front and the rear of the diaper.

The width of the absorbent assembly in direction Y is preferably 35% to 65%, more preferably 40% to 60%, of the width in direction Y of the front and rear panel 2A and 2B in the state shown in Fig. 2 or the maximum stretched length of the front and rear panel 2A and 2B in that direction.

The outer sheet 22 and the inner sheet 23 may be of any sheet materials conventionally used in this type of articles but are preferably formed of nonwoven fabric. In view of softness and the like, a single sheet or a laminate of two or more sheets of nonwoven fabric, such as air-through nonwoven, heat-rolled nonwoven, hydroentangled nonwoven, spun bonded nonwoven, and melt blown nonwoven fabrics, is particularly preferred. A nonwoven fabric integrally laminated with film is also useful. Molding materials making the elastic members 24, 36, and 39 are not particularly limited and may be any of known elastic materials of various kinds that have been used in absorbent articles, such as disposable diapers and sanitary napkins. Examples of such elastic materials include synthetic rubbers, such as styrene-butadiene, butadiene, isoprene, and neoprene, natural rubber, EVA, stretch polyolefins, and polyurethane. Forms of the elastic members preferably include a thread with a rectangular, square, circular or polygonal section, tape, or multifilamentous yarn.

A second and a third embodiment of the invention will then be described.

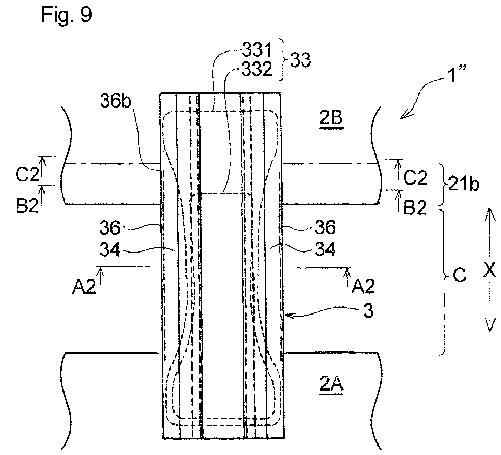

Description of the disposable pull-on diapers of the second and the third embodiment will generally be confined to the differences from the first embodiment, with the details in common omitted. The description of the first embodiment applies to the second and the third embodiment with the exceptions noted hereunder. The elements or members shown in Figs. 7 through 10 that are common to the first embodiment are identified with the same reference numerals as in the first embodiment.

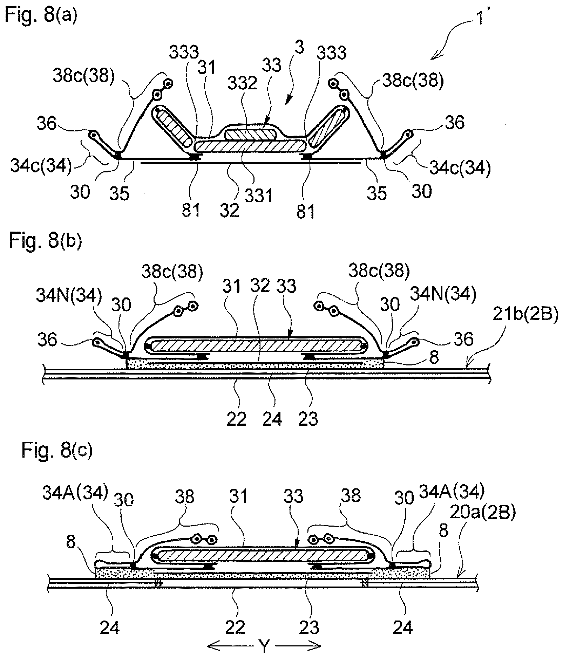

Figs. 8(a) to 8(c) depict a diaper 1' according to the second embodiment, in which the topsheet 31 wraps the side edges and the underside of the absorbent member 33 and is fastened to the side gather-forming sheet 35 on the side of the backsheet 31 along a position laterally inward from each side edge of the absorbent member 33 to form a bond 81. The absorbent member 33 in the diaper 1' has a lower absorbent core 331, an upper absorbent core 332, and an unshown core wrap sheet wrapping them. The lower absorbent core 331 is rectangular in a plan view and has a laterally opposite pair of flex-inducing regions 333 in its area located in the crotch portion C. The upper absorbent core 332 is smaller than the lower absorbent core 331 and superposed on the lower absorbent core 331. The flex-inducing regions 333 may be a cutout region (a region where a core-forming material which forms the absorbent core, such as pulp fiber, is missing), a low weight region (a region having a lower basis weight of the core-forming material than other region), a slit, and the like that serves to reduce the bending stiffness to help both side edges of the absorbent member 33 to rise upward.

Similarly to the first embodiment, the diaper 1' of the second embodiment has side sheet portions 34 and standing gather-forming portions 38 that form side gathers 34c and standing gathers 38c, respectively, in the crotch portion C. A linear bond 30 that provides a base of rising of these gathers is formed by bonding a doubled-over portion of the sheet 35.

Each side sheet portion 34 in the diaper 1' has a bonded region 34A and a non-bonded region 34N. The bonded region 34A is located on the rear panel 2B nearer the longitudinal end 3b of the absorbent assembly 3 and is fixed in its laterally outwardly lying position to the rear panel 2B as shown in Fig. 8(c). The non-bonded region 34N is located on the rear panel 2B nearer the crotch portion C and is not bonded to the rear extension portion 21b as shown in Fig. 8(b). Each side sheet portion 34 is elasticized in the non-bonded region 34N, too.

Therefore, the diaper 1' of the second embodiment produces the same effects as those discussed for the first embodiment.

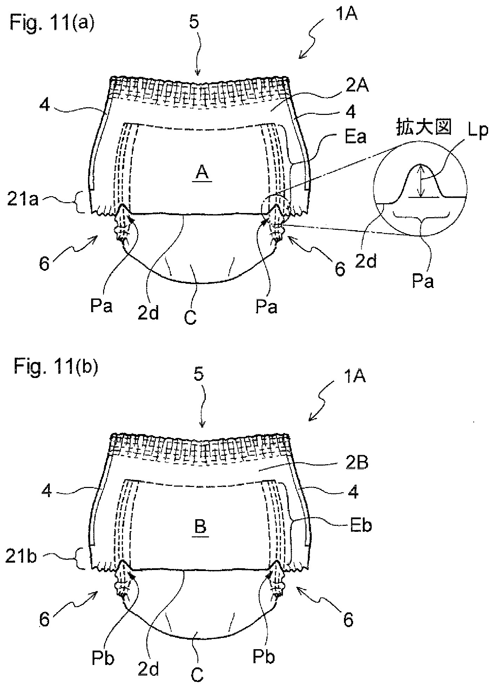

Figs. 11 and 12 depict a disposable pull-on diaper 1A according to a fourth embodiment of the invention (hereinafter simply "diaper A"). As shown, the diaper 1A includes a front panel 2A adapted to be worn about the wearer's front and having opposite lateral side edges 2a and 2a, a rear panel 2B adapted to be worn about the wearer's rear and having opposite lateral side edges 2b and 2b, and an absorbent assembly 3 fixed to the front panel 2A and the rear panel 2B so as to bridge them. The lateral side edges 2a and 2a of the front panel 2A and the lateral side edges 2b and 2b of the rear panel 2B are joined together, respectively, to form a pair of opposite side seals 4 and 4.

The front panel 2A and the rear panel 2B have a length La and Lb, respectively, both greater than the length L4 of the side seals 4 in the longitudinal direction (direction X) of the diaper (i.e., absorbent article). That is, the front panel 2A has a front extension portion 21a extending downward (while worn) from the side seals 4, and the rear panel 2B has a rear extension portion 21b extending downward (while worn) from the side seals 4. The portion of the front panel 2A that is located between the opposite side seals 4 and 4 (the portion other than the front extension portion 21a) is called the front panel main portion 20a. The portion of the rear panel 2B that is located between the opposite side seals 4 and 4 (the portion other than the front extension portion 21b) is called the rear panel main portion 20b.

Each of the front panel 2A and the rear panel 2B is oblong rectangular as shown in Fig. 12.

The term "upward" and the term "downward" as used while the absorbent article is worn mean toward the side of the waist opening and toward the side of the crotch portion, respectively, in Fig. 11.

More specifically, as shown in Figs. 11 and 12, the diaper 1A has a front portion A worn about the wearer's front, a rear portion B worn about the wearer's rear, and the crotch portion C located between the front portion A and the rear portion B and worn about the wearer's crotch while worn. The diaper 1A (absorbent article) has a longitudinal direction (direction X in Fig. 12) extending from the front portion A through the crotch portion C to the rear portion B or vice versa and a lateral direction (direction Y in Fig. 12) perpendicular to the longitudinal direction.

The absorbent assembly 3 of the diaper 1A has a rectangular shape longer in direction X as shown in Fig. 12. As shown in Fig. 13, the absorbent assembly 3 includes a liquid permeable topsheet 31, a liquid impermeable or water repellent backsheet 32, and a liquid retentive absorbent member 33 interposed between the two sheets 31 and 32. The absorbent member 33 is composed of an absorbent core 33a formed of an aggregate of fibers (e.g., pulp fiber), which may be nonwoven fabric, with or without a superabsorbent polymer incorporated therein, and core wrap sheets 33b and 33c wrapping the absorbent core 33a. The absorbent member 33 is also rectangular, longer in direction X. The absorbent member 33 and the backsheet 32 are bonded by any means, such as application of an adhesive, heat sealing, or ultrasonic sealing.

The absorbent assembly 3 has a pair of opposite side sheet portions 34 and 34 along both lateral sides thereof.

As shown in Fig. 12, the side sheet portion 34 extends in the longitudinal direction of the absorbent assembly 3 over the whole length of the absorbent assembly 3. As shown in Fig. 13, each side sheet portion 34 includes a liquid resistant or water repellent sheet 35 and elastic members 36 and 36 fixed to the sheet 35.

Substantially the entire area of the part of each side sheet portion 34 where the elastic members 36 are disposed in the longitudinal direction of each side sheet portion 34 provides an elasticized region 134c in which the elastic members are fixed in their stretched state in the longitudinal direction of the absorbent assembly. The elasticized region 134c is elasticized in the longitudinal direction of the absorbent assembly 3.

The elasticized region 134c of each side sheet portion 34 forms a side gather in the crotch portion C (i.e., the portion extending in the longitudinal direction (direction x) of the absorbent assembly between the front panel 2A and the rear panel 2B).

That is, on contraction of the elastic members 36 fixed in their stretched state in the side sheet portion 34, the elasticized region 134c in the crotch portion C forms the side gather as a result of gathering or deformation of the sheet 35 forming the side sheet portion 34 into a wavy cross-section. It suffices that the side gather be formed while the diaper is in a relaxed state and/or during wear. While worn, the side gather rises toward the wearer's body starting from the distal edge of a linear bond 30 (hereinafter described) as a rising base 34b.

As shown in Fig. 12, the elasticized region 134c of the side sheet portion 34 also extends on the rear panel 2B and bonded to the rear panel 2B. The part of the rear panel 2B to which the elasticized region 134c is bonded is a rear longitudinally elasticized region Eb that extends and contracts together with the elasticized region 134c.

As shown in Fig. 11(b), in a relaxed state of the diaper 1A with the rear longitudinally elasticized region Eb contracted, the lower edge 2d of the rear panel 2B near each rear longitudinally elasticized region Eb forms downward concaves Pb, Pb.

In the present embodiment, the elasticized region 134c of the side sheet portion 34 also extends on the front panel 2A and bonded to the front panel 2A. The part of the front panel 2A to which the elasticized region 134c is bonded is a front longitudinally elasticized region Ea that extends and contracts together with the elasticized region 134c.

As shown in Fig. 11(a), in a relaxed state of the diaper 1A with the front longitudinally elasticized region Ea contracted, the lower edge 2d of the front panel 2A near each front longitudinally elasticized region Ea has downward concaves Pa, Pa.

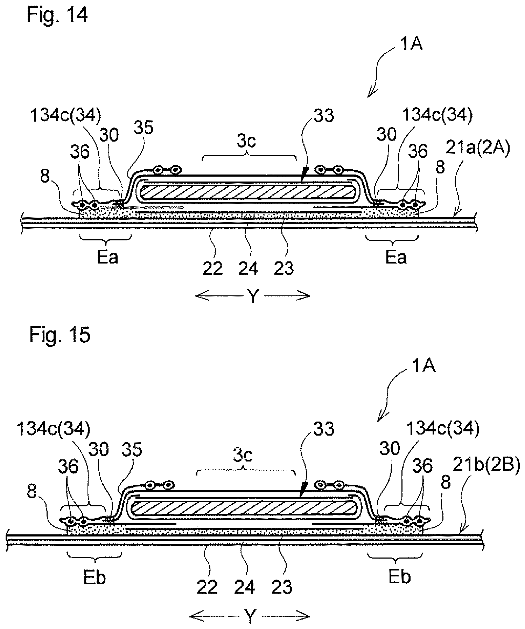

The diaper 1A of the present embodiment will be described in greater detail. As shown in Fig. 12, the front side end of each elastic member 36 is at almost the same position of the front side end 3a of the absorbent assembly 3, so that the elastic members 36 and the elasticized region 134c of each side sheet portion 34 exhibit extensibility and contractibility over the entire length (in direction X) of its region overlapping the front panel 2A. Substantially the entire area of the region of the side sheet portion 34 that overlaps the front panel 2A is bonded to the front panel 2A with an adhesive 8 as shown in Fig. 14. The entire length (in direction X) of the region of the front panel 2A with which the side sheet portion 34 overlaps is the front longitudinally elasticized region Ea.

The rear side end of each elastic member 36 is at almost the same position of the rear side end 3b of the absorbent assembly 3, so that the elastic members 36 and the elasticized region 134c of each side sheet portion 34 exhibit extensibility and contractibility in the entire length (in direction X) of its region overlapping the rear panel 2B. Substantially the entire area of the region of the side sheet portion 34 that overlaps the rear panel 2B is bonded to the rear panel 2B with an adhesive 8 as shown in Fig. 15. The entire length (in direction X) of the region of the rear panel 2B with which the side sheet portion 34 overlaps is the rear longitudinally elasticized region Eb.

As shown in Fig. 14, the elastic members 36 in the front longitudinally elasticized region Ea are fixed by means, e.g., of an unshown adhesive between two layers of the sheet 35 forming the side sheet portion 34, and the side sheet portion 34 having the elastic members 36 thus fixed inside thereof is fixed to the front panel 2A via an adhesive 8. The same configuration applies to the elastic members 36 forming the rear longitudinally elasticized region Eb. That is, as shown in Fig. 15, the elastic members 36 are fixed by means, e.g., of an unshown adhesive between two layers of the sheet 35 forming the side sheet portion 34, and the side sheet portion having the elastic members 36 thus fixed inside thereof is fired to the rear panel 2B with an adhesive 8.

While the elastic members 36 are preferably disposed in parallel with direction X as shown in Fig. 12, they may be somewhat angled with respect to direction X or curved laterally inwardly or outwardly.

Since the diaper 1A of the present embodiment has the rear extension portion 21b in the rear panel 2B, the position of the lower edge 2d of the rear panel 2B on the wearer's body is lower than with a conventional diaper in which both side edges of the front panel 2A and both side edges of the rear panel 2B are joined together over their total length in the diaper longitudinal direction. In addition, the rear longitudinally elasticized regions Eb of the rear panel 2B serve to lift the lower edge 2d of the rear panel 2B at and near the sides of the absorbent assembly 3 while the diaper 1A is worn. As a result, the position of the lower edge 2d of the rear panel 2B is prevented from largely differing between the side of the absorbent assembly 3 and the side of the side edge 2b, whereby an improved coverage over the buttocks is provided.

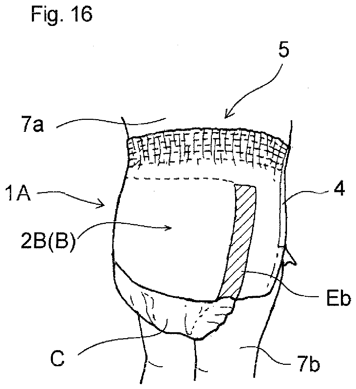

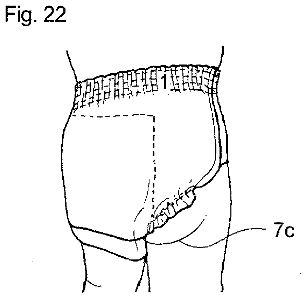

Fig. 16 illustrates a waist opening 5 and leg openings 6 and 6 through which the torso 7a and legs 7b of a child extend, respectively. Fig. 22 depicts a conventional pull-absorbent article having a rear extension portion, in which the lower edge 2d of the rear panel is positioned considerably above the line 7c appearing between each buttock and thigh of a wearer.

To further ensure the above described effects, it is preferred that the length L2' (see Fig. 12) of the part of the rear longitudinally elasticized region Eb that exists in the rear extension portion 21b is 40% to 100%, more preferably 70% to 100%, even more preferably 90% to 100%, of the length L6 (see Fig. 12) of the rear extension portion 21b.

It is preferred that the rear longitudinally elasticized region Eb be formed over a length including at least half of the elastic members 24 disposed in the elasticized extension region G3, more preferably over a length including all the elastic members 24 disposed in the elasticized extension region G3 except the closest one to the crotch portion, even more preferably over a length including all the elastic members 24 disposed in the elasticized extension region G3. The same preference also applies to the front longitudinally elasticized region Ea as hereinafter described.

For the same purpose, it is preferred that the rear longitudinally elasticized region Eb be formed over a length straddling the rear extension portion 21b and the rear panel main portion 20b as shown in Fig. 12.

In the relaxed state of the diaper 1A, the depth Lp of the concave Pb (see Fig. 11(a)) formed of the lower edge 2d of the rear panel 2B is preferably 15% to 75%, more preferably 30% to 70%, of the length L6 of the rear extension portion 21B.

The diaper 1A of the present embodiment has a pair of standing gather-forming portions 38 extending along both sides of the absorbent assembly 3 in the longitudinal direction of the absorbent assembly 3.

The configuration of the standing gather-forming portions 38 and the linear joints 30 and the effects of the standing gathers 38c are the same as those discussed for the first embodiment, and the description about them given with respect to the first embodiment applies to the present embodiment as appropriate.

Since the standing gather 38 is provided laterally inwardly from the side sheet portion 34, if a bodily fluid leaks through a gap formed between the diaper 1A and the wearer's body with the movement of the body, the gathers standing in different directions will provide a reliable double protection to prevent such a lateral leakage through the gap. The standing gather 38c forms in at least the relaxed state of the diaper 1A.

In a flat-out, uncontracted state of the diaper 1A (see Fig. 12), the front panel 2A has a rectangular shape longer in the lateral direction and has opposite lateral side edges 2a and 2a extending in the diaper longitudinal direction (direction X) and opposite longitudinal end edges 2c and 2d extending in the diaper lateral direction (direction Y). Similarly, the rear panel 2B has a rectangular shape longer in the lateral direction, opposite lateral side edges 2b and 2b extending in the diaper longitudinal direction (direction X), and opposite longitudinal end edges 2c and 2d extending in the diaper lateral direction (direction Y). As depicted in Fig. 12, the length of each of the front panel 2A and the rear panel 2B in the diaper longitudinal direction (direction X) is uniform in the diaper lateral direction (direction Y).

Each of the side edges 2a of the front panel 2A has a joined region 2a' where the front panel 2A and the rear panel 2B are joined together to form a side seal 4 and a non-joined region 2a" which extends downward from the joined region 2a' and in which the front panel 2A and the rear panel 2B are not joined together. Similarly, each of the side edges 2b of the rear panel 2B has a joined region 2b' in which the front panel 2A and the rear panel 2B are joined together to form a side seal 4 and a non-joined region 2b" which extends downward from the joined region 2b' and in which the front panel 2A and the rear panel 2B are not joined together. The non-joined region 2a" is on the extended line of the joined region 2a', and the non-joined region 2b" is on the extended line of the joined region 2b', as shown in Fig. 12.

The joined region 2a', which is a part of the side edge 2a of the front panel 2A, and the joined region 2b', which is a part of the side edge 2b of the rear panel 2B, on each side of the diaper 1A are joined to each other to form a pair of the side seals 4 and 4, a waist opening 5, and a pair of leg openings 6 and 6. Joining the front and the rear panel is achieved by, for example, heat sealing, high frequency sealing, ultrasonic sealing, or adhesive application.

The joined region 2a' and non-joined region 2a" of each side edge 2a of the front panel 2A connect to each other and extend in the diaper longitudinal direction in the flat-out uncontracted state of the diaper (see Fig. 12). The joined region 2b' and non-joined region 2b" of each side edge 2b of the rear panel 2B also connect to each other and extend in the diaper longitudinal direction in the flat-out uncontracted state of the diaper (see Fig. 12). Fig. 11 illustrates the waist opening 5 and the leg openings 6 and 6 through which the torso 7a and legs 7b of a child extend, respectively.

The front and the rear panel 2A and 2B of the diaper 1A each include an outer sheet 22 that defines the exterior of the diaper, an inner sheet 23 laid on the inner side of the outer sheet 22, and a plurality of elastic members 24 of thread form spacedly arranged to extend in direction X between the two sheets 22 and 23 as shown in Figs. 14 and 15. Thus, the front and the rear panel 2A and 2B each have an elasticized waist region G1, an elasticized below-waist region G2, and an elasticized extension region G3 as shown in Fig. 12.

In embodiments in which the front and the rear panel 2A and 2B are elasticized by the elastic members 24 disposed between the two sheets 22 and 23 in their stretched state, it is preferred for the opposite side edges 2a and 2a and for the opposite side edges 2b and 2b to be parallel with each other with the elastic members 24 in their stretched state as shown in Fig. 12. Each of the front and the rear panel 2A and 2B is preferably rectangular longer in the diaper lateral direction with the elastic members 24 in their stretched state.

The elasticized waist region G1 is outward from the longitudinal end 3a or 3b of the absorbent assembly 3 in the diaper longitudinal direction (direction X) in each of the front and the rear panels 2A and 2B. The elasticized below-waist region G2 is between the elasticized waist region G1 and the extension portion 21a or 21b along direction X in each of the front and the rear panel 2A and 2B. The elasticized extension region G3 is formed in the front extension portion 21a and in the rear extension portion 21b. It is preferred that each of the elasticized below-waist region G2 and the elasticized extension region G3 be formed in at least a region laterally outward from each lateral side edge of the absorbent assembly 3.

It is more preferred that each of the elasticized waist region G1, elasticized below-waist region G2, and elasticized extension region G3 be elasticized in at least a region laterally outward from each lateral side edge of the absorbent member 33.

While the embodiment shown in Figs. 11 through 15 has the elastic members 24 extending over the whole width of the front and the rear panel 2A and 2B across the absorbent member 33 to form the waist elasticized region G1, elasticized below-waist region G2, and elasticized extension region G3, it is preferred that the elastic members 24 forming the elasticized below-waist region G2 and/or the elasticized extension region G3 be deelasticized by heating, cutting, or like means in an area overlapping a laterally middle portion of the absorbent member 33 so as not to elasticize that part of the front and rear panel 2A and 2B.

The two sheets 22 and 23 making up the front and rear panels 2A and 2B may be bonded to each other with an adhesive over substantially the entire area thereof or, as with the case of the elasticized laminate sheet described in JP 2005-80859A, may be bonded at a large number of bonds discretely arranged therebetween in a pattern that does not overlap the elastic members 24.

The absorbent assembly 3 is fixed at one longitudinal end portion thereof (the portion overlapping the front panel 2A) to the lateral (direction Y) middle portion of the front panel 2A with an adhesive 8, and is fixed at the opposite end portion (the portion overlapping the rear panel 2B) to the lateral (direction Y) middle portion of the rear panel 2B with an adhesive 8.

In the diaper 1A of the present embodiment, each side sheet portion 34 is fixed in its laterally (direction Y) outwardly lying position to the front panel main portion 20a and the front extension portion 21a of the front panel 2A via an adhesive 8 as shown in Figs. 12 and 14. Each side sheet portion 34 is also fixed in its laterally (direction Y) outwardly lying position to the rear panel main portion 20b and the rear extension portion 21b of the rear panel 2B via an adhesive 8 as shown in Figs. 12 and 15.

The absorbent assembly 3 preferably has its lateral middle portion 3c bonded to the rear panel main portion 20b and the rear extension portion 21b by means of, for example, an adhesive 8. By this bonding, the rear panel 2B is prevented from separating from the absorbent assembly 3, which raises the perfection level of aesthetic appearance. Furthermore, the adhesive bonding improves stiffness, which is effective to maintain the width of the absorbent core and thereby to prevent leakage.

Since the diaper 1A of the present embodiment has a front extension portion 21a in the front panel 2A, it has a good appearance from the side of the front portion A without looking like loincloth.

As previously stated, the front panel 2A has the front longitudinally elasticized regions Ea. The front longitudinally elasticized regions Ea serve to lift the lower edge 2d of the front panel 2A at and near the sides of the absorbent assembly 3 while the diaper 1A is worn. As a result, the lower edge 2d of the front panel 2A is maintained relatively horizontal to provide a good appearance from the side of the front portion A of the diaper 1A. While the front extension portion 21a is not essential to the diaper of the invention, the configuration of the diaper 1A of the present embodiment, in which the front panel 2A has a front extension portion 21a, and the elasticized region 134c of the side sheet portion is bonded to the front extension portion 21a to form the front longitudinally elasticized region Ea, is preferred from the viewpoint of improved appearance from the side of the front portion A.

It is preferred that the length L1 (see Fig. 12) of the part of the front longitudinally elasticized region Ea that is located in the front extension portion 21a is 40% to 100%, more preferably 70% to 100%, even more preferably 90% to 100%, of the length L5 (see Fig. 12) of the front extension portion 21 a.

It is preferred from the same viewpoint that the front longitudinally elasticized region Ea be formed to straddle the front extension portion 21a and the front panel main portion 20a.

A preferred range of the depth Lp of the concave Pa formed of the lower edge 2d of the front panel 2A is the same as that described with respect to the concave Pb.

Because the elasticized extension region G3 formed in each of the front extension portion 21a and the rear extension portion 21b contracts to a moderate degree by the elastic members 24 disposed therein, when the diaper 1A is fitted to a wearer, the side edge 2a of the front extension portion 21a of the front panel 2A and the side edge 2b of the rear extension portion 21b of the rear panel 2B on each side of the diaper 1A separate from each other toward the front and the rear in the shape of an inverted letter Y. Therefore, the diaper 1A has a good appearance from the side and looks like providing good protection against leakage.

Because the front extension portion 21a of the front panel 2A forms the elasticized extension region G3 that extends and contracts in the diaper lateral direction, the front extension portion 21a comes into planar contact to the skin to provide coverage on the groins. After urination, the increased weight is supported by the front extension portion 21a, the front panel main portion 20a above the front extension portion 21a, and the rear panel main portion 20b. As a result, the pressure is diffused to prevent leaving red marks.

The diaper 1A is produced effectively by a method including advancing a continuous front panel 2A and a continuous rear panel 2B at a predetermined distance therebetween, fixing adsorbent assemblies 3 at intervals to the continuous front panel 2A and the continuous rear panel 2B so as to bridge them, folding the resulting continuous form of diaper so that the continuous front panel 2A and the continuous rear panel 2B face each other, forming side seals 4 by heat sealing or like means (joining), and cutting the continuous form of diaper into individual disposable diapers simultaneously or after the joining. This method offers the advantage over a method involving making through-holes or cutouts for forming leg openings that the need to trim a continuous length of outer cover material is eliminated, or the size of unnecessary parts to be trimmed off is reduced. In order to eliminate the need to trim, it is particularly preferred that the continuous front panel 2A and the continuous rear panel 2B be cut along a straight line perpendicular to the machine direction to become the front panel 2A and the rear panel 2B, respectively, of a finished diaper 1A.

Fifth to seventh embodiments of the invention will then be described.

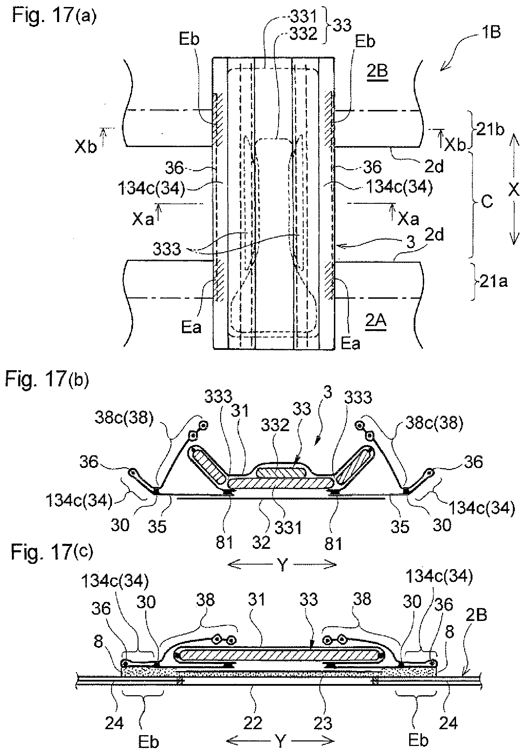

Description of the disposable pull-on diapers of the fifth to seventh embodiments will generally be confined to the differences from the fourth embodiment, with the details in common omitted. The description of the fourth embodiment applies to the fifth to seventh embodiments with the exceptions noted hereunder. The elements or members shown in Figs. 17 through 21 that are common to the fourth embodiment are identified with the same reference numerals as in the fourth embodiment.

A diaper 1B according to the fifth embodiment has the topsheet 31 thereof wrapping the side edges and the underside of the absorbent member 33 and fastened to the sheet 35 (forming the side sheet portion 34) on the side of the backsheet 31 along a position laterally inward from each side edge of the absorbent member 33 to form a bond 81. The absorbent member 33 in the diaper 1B has a lower absorbent core 331, an upper absorbent core 332, and an unshown core wrap sheet wrapping them. The lower absorbent core 331 is rectangular in a plan view and has a laterally opposite pair of flex-inducing regions 333 in the area corresponding to the crotch portion C. The upper absorbent core 332 is smaller than the lower absorbent core 331 and placed on the lower absorbent core 331. Each flex-inducing region 333 may be a cutout region (a region where the core-forming material, such as pulp fiber, is missing), a low weight region (a region having a lower basis weight of the core-forming material than others), a slit, and the like that serves to reduce the bending stiffness to help both side edges of the absorbent member 33 to rise upward.

Similarly to the fourth embodiment, the diaper 1B of the fifth embodiment has side sheet portions 34 and standing gather-forming portions 38 that form side gathers and standing gathers 38c, respectively, in the crotch portion C. A linear bond 30 that provides a base of rising of these gathers is formed by bonding a doubled-over portion of the sheet 35.

In the diaper 1B of the fifth embodiment, too, each side sheet portion 34 is fixed in its laterally outwardly lying position to the front panel 2A and the rear panel 2B as shown in Fig. 17(c).

Specifically, as shown in Fig. 17(a), the side sheet portion 34 having an elastic member 36 fixed therein in its stretched state is bonded to the rear extension portion 21b and the front extension portion 21a to form a rear longitudinally elasticized region Eb and a front longitudinally elasticized region Ea, respectively. As a result, in a relaxed state of the diaper 1B, the lower edge of the rear panel 2B forms an unshown downward concave similar to the concave Pb shown in Fig. 11, and the lower edge of the front panel 2A forms an unshown downward concave similar to the concave Pa shown in Fig. 11. Accordingly, the diaper 1B of the fifth embodiment produces the same effects as those discussed for the fourth embodiment.

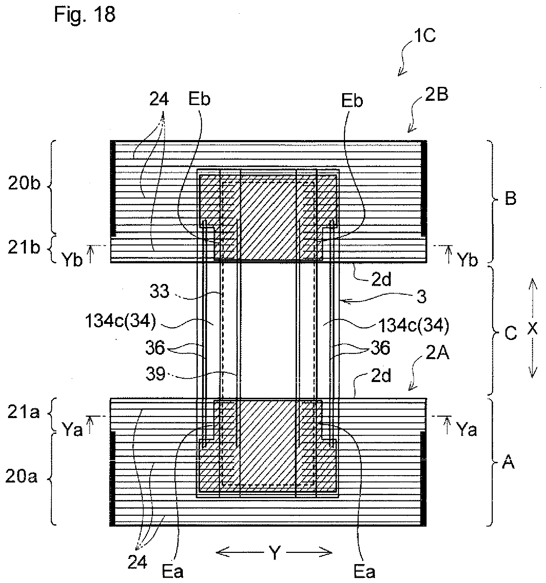

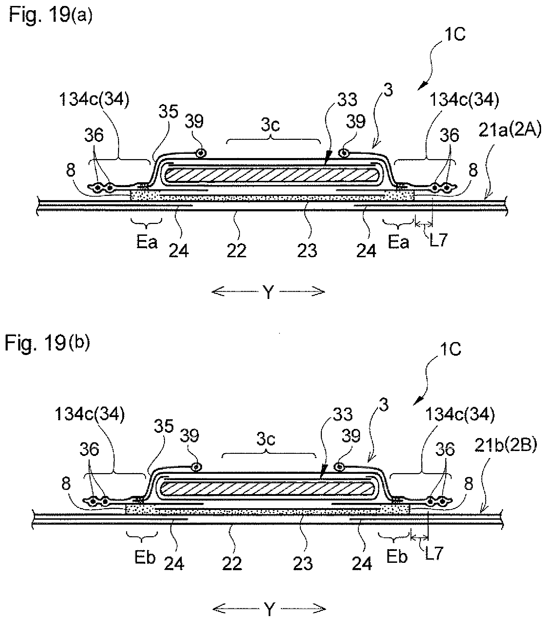

A disposable pull-on diaper 1C according to the sixth embodiment (see Figs. 18 and 19) is different from the fourth embodiment in the geometry of bonding the elasticized region 134c of the side sheet portion 34 to the rear panel 2B and the front panel 2A. Specifically, in the diaper 1A of the fourth embodiment the elasticized region 134c of each side sheet portion 34 is bonded to the rear extension portion 21b of the rear panel 2B over its whole width (in direction Y) including the part where the elastic members 36 are disposed as shown in Fig. 15. In the diaper 1C of the sixth embodiment, on the other hand, the elasticized region 134c is bonded with the adhesive 8 to the rear extension portion 21b over only a part of its width (in direction Y) nearer the absorbent body 33, with the part where the elastic members 36 are disposed not being bonded to the rear extension portion 21b, as shown in Fig. 19(b).

Likewise, in the diaper 1A of the fourth embodiment the elasticized region 134c of each side sheet portion 34 is bonded to the front extension portion 21a of the front panel 2A over its whole width (in direction Y) including the part where the elastic members 36 are disposed as shown in Fig. 14. In the diaper 1C of the sixth embodiment, on the other hand, the elasticized region 134c is bonded with the adhesive 8 to the front extension portion 21a over only a part of its width (in direction Y) nearer the absorbent body 33, with the part where the elastic members 36 are disposed not being bonded to the front extension portion 21a, as shown in Fig. 19(a).

The shaded regions in Fig. 18 are the regions where the absorbent assembly 3 is bonded to the rear panel 2B and the front panel 2A in the diaper 1C of the sixth embodiment.

In the sixth embodiment, the elasticized region 134c of each side sheet portion 34 is fixed to the rear extension portion 21b over a part of its width (in direction Y) to form a rear longitudinally elasticized region Eb in the rear panel 2B. The rear longitudinally elasticized region Eb is elasticized in the longitudinal direction of the absorbent assembly 3 (direction X). The rear longitudinally elasticized region Eb therefore contracts in a relaxed state of the diaper to cause the lower edge 2d of the rear panel 2B to form an unshown downward concave at and near the rear longitudinally elasticized region Eb.

Similarly, the elasticized region 134c of each side sheet portion 34 is fixed to the front extension portion 21a over a part of its width (in direction Y) to form a front longitudinally elasticized region Ea. The front longitudinally elasticized region Ea contracts in a relaxed state of the diaper to cause the lower edge 2d of the front panel 2A to form an unshown downward concave at and near the rear longitudinally elasticized region Ea.

The diaper 1C of the sixth embodiment therefore exhibits the similar but slightly weaker effects to/than those obtained in the fourth embodiment.

The distance L7 (see Figs. 19(a) and 19(b)) in the lateral direction (direction Y) of the absorbent assembly 3 between the part in which the elasticized region 134c is bonded to the front or rear extension portion 21a or 21b and the elastic members 36 fixed in the elasticized region 134c may be, for example, more than 0 mm and not more than 10 mm. The distance L7 may be, for example, 50% to 80% of the sum of the width (in direction Y) of the front or rear longitudinally elasticized region Ea or Eb and the length L7.

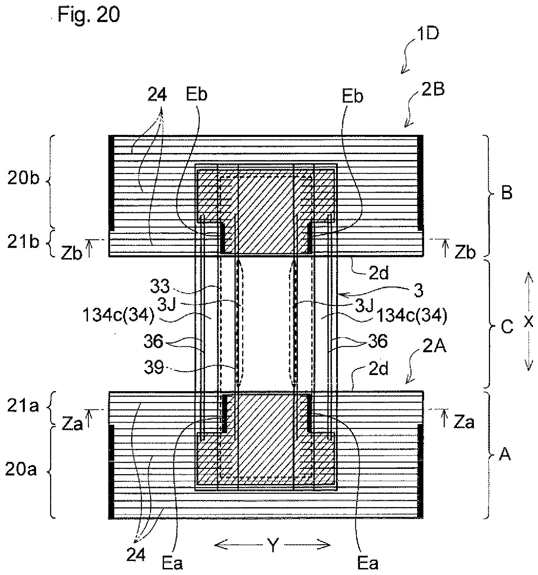

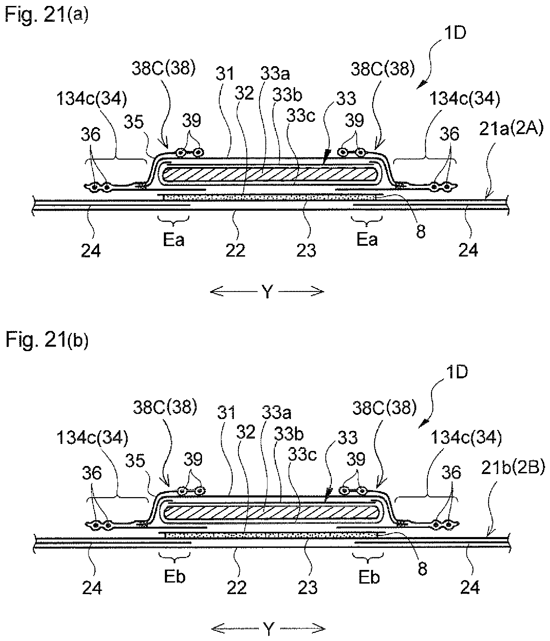

In Figs. 20 and 21 is shown a disposable pull-on diaper 1D according to the seventh embodiment. As shown, the absorbent assembly 3 has an oblong absorbent body 33, a pair of side sheet portions 34, and a pair of standing gather-forming portion 38 similarly to the fourth embodiment. The side sheet portions 34 are located laterally outwardly from the side edges of the absorbent member 33 and have an elasticized region 134c in the crotch portion C and on the rear extension portion. Each elasticized region 134c is elasticized in direction X by the elastic members 36 fixed in their stretched state between two layers of the sheet 35 forming each side sheet portion 34. As shown in Fig. 21(b), a part of each elasticized region 134c in its width direction (direction Y) where the elastic members 36 are disposed is not bonded to the rear extension portion 21b. More specifically, the elasticized region 134c is not bonded to the rear extension portion 21b in its region laterally outward from the side edge of the absorbent member 33.

The shaded regions in Fig. 20 are the regions where the absorbent assembly 3 is bonded to the rear panel 2B and the front panel 2A in the diaper 1D of the seventh embodiment.

As shown in Fig. 20, each side sheet portion 34 is fixed in its laterally outwardly lying position to the front panel 2A and the rear panel 2B in its opposite longitudinal end portions. Each of the standing gather-forming portions 38, on the other hand, is fixed in its laterally inwardly lying position to the topsheet 31 in its opposite longitudinal end portions.

Each standing gather-forming portion 38 has elastic members 39 fixed in their stretched state over the length from the front extension portion 21a to the rear extension portion 21b, whereby a standing gather 38c is formed to extend through the crotch portion C and straddle the front extension portion 21 a and the rear extension portion 21b. While the diaper is worn, the standing gather 38c stands with its edge having the elastic members 39 up by the contractive force of the elastic members 39.

The diaper 1D of the seventh embodiment is designed such that the contractive force of the standing gathers 38c formed on the rear extension portion 21b is exerted to, or to near, the respective side edges of the absorbent member 33, whereby at least the rear extension portion 21b of the rear panel 2B forms a rear longitudinally elasticized region Eb having extensibility and contractibility in the longitudinal direction of the absorbent assembly 3 (direction X) near each side edge of the absorbent member 33. In a relaxed state of the diaper with the rear longitudinally elasticized region Eb contracted, the lower edge 2d of the rear panel 2B has a downward concave (not shown) near the rear longitudinally elasticized region Eb.

The diaper 1D of the seventh embodiment is also designed to form a pair of standing gather 38c on the front extension portion 21a such that the contractive force of the standing gathers 38c formed on the rear extension portion 21b is exerted to, or to near, the respective side edges of the absorbent member 33. As a result, at least the front extension portion 21b of the front panel 2A forms a front longitudinally elasticized region Ea having extensibility and contractibility in the longitudinal direction of the absorbent assembly 3 (direction X) near each side edge of the absorbent member 33. In a relaxed state of the diaper with the front longitudinally elasticized region Ea contracted, the lower edge 2d of the front panel 2A has a downward concave (not shown) near the front longitudinally elasticized region Ea.

The diaper 1D of the seventh embodiment therefore exhibits the similar but slightly weaker effects to/than those produced by the diaper 1 of the fourth embodiment.

To ensure formation of the downward concave along the lower edge 2d of the rear panel 2B near the rear longitudinally elasticized region Eb, it is preferred for the elasticized region 134c of each side sheet portion 34 to straddle the crotch portion C and the rear extension portion 21b. To ensure formation of the downward concave along the lower edge 2d of near the front longitudinally elasticized region Ea, it is preferred for the elasticized region 134c of the side sheet portion 34 to extend in the crotch portion C and on the front extension portion 21a.

To ensure formation of the downward concave along the lower edge 2d of the front panel 2A near the rear longitudinally elasticized region Eb, it is preferred for the elasticized region 134c of the side sheet portion 34 to extend in the crotch portion C and on the rear extension portion 21b. To ensure formation of the downward concave along the lower edge 2d near the front longitudinally elasticized region Ea, it is preferred for the elasticized region 134c of the side sheet portion 34 to extend in the crotch portion C and on the front extension portion 21a.

To ensure formation of the downward concave along the lower edge 2d of the rear panel 2B and/or the front panel 2A, it is preferred for the absorbent member 33 to have a flexing region 3J, such as a cutout region (a region where the core-forming material which forms an absorbent core 33a, such as pulp fiber, is missing) or a low weight region (a region having a lower basis weight of the core-forming material which forms the absorbent core 33a than other region), between the central core segment and each side core segment in the crotch portion C as shown in Fig. 20. The flexing regions 3J help the side core segments of the absorbent member 33 to contract independently of the central core segment. As a result, contraction of the longitudinally elasticized regions is not hindered, and the downward concaves are readily formed along the lower edge 2d of the rear panel 2B and/or the front panel 2A.

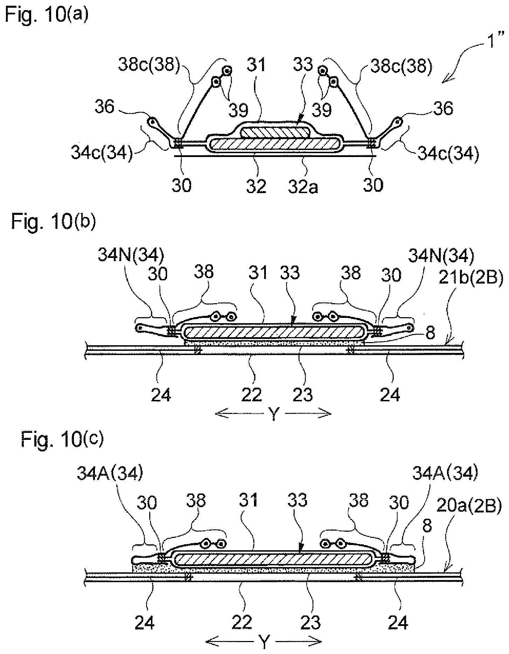

Fig. 10 illustrates a diaper 1" according to the third embodiment. In the third embodiment, the topsheet 31 and the backsheet 32 are bonded together outboard of the absorbent member 33. The bond between the topsheet 31 and the backsheet 32 is sandwiched between and fixed to two layers of the doubled sheet 35 to form a linear bond 30. The absorbent member 33 of the diaper 1" is composed of an hour glass-shaped lower absorbent core 331, an upper absorbent core 332 smaller than the lower absorbent core 331 and placed on the lower absorbent core 331, and an unshown core wrap sheet that wraps these absorbent cores.

Similarly to the first embodiment, the diaper 1" of the third embodiment has a pair of side sheet portions 34 and a pair of standing gather-forming portions 38 that form a pair of side gathers 34c and a pair of standing gathers 38c, respectively, in the crotch portion C.

In the third embodiment, each side sheet portion 34 of the diaper 1" has a bonded region 34A where the side sheet portion 34 is fixed in its laterally outwardly lying position to the rear panel 2B in a portion thereof overlapping the rear panel 2B and nearer the end 3b of the absorbent assembly 3 as shown in Fig. 10(c). Each side sheet portion 34 also has a non-bonded region 34N where the side sheet portion 34 is not fixed to the rear extension portion 21b in a portion thereof overlapping the rear panel 2B and nearer the crotch portion C. The side sheet portion 34 is elasticized in the non-bonded region 34N as well.

Accordingly, the diaper 1" of the third embodiment produces the same effects as those discussed for the first embodiment.

While the invention has been described with reference to its preferred embodiments, it should be understood that the invention is not limited thereto, and various changes and modifications can be made therein as follows.

The absorbent assembly 3 may dispense with the standing gather-forming portions.

The elasticized below-waist region G2 may span the whole width of the front panel 2A and/or the rear panel 2B. The elasticized extension region G3 may also span the whole width of the front panel 2A and/or the rear panel 2B.

The front extension portion 21a may be dispensed with. The front extension portion 21a may dispense with the elastic members 24.

The sheet forming the side sheet portion 34 and the sheet forming the standing gather-forming portion may be separate sheets. Each of these sheets may be a single-layered sheet, which is partly folded upon itself to form a plurality of layers, between which an elastic member is fixed.

While each side seal 4 is formed by bonding the side edges 2a and 2b of the front and rear panel, there may be a non-bonded region outboard of the side seal 4, in which the front panel 2A and the rear panel 2B are not bonded together over a small width, e.g., more than 0 mm and not more than 20 mm.

The pull-on absorbent articles of the invention (e.g., the diapers 1, 1', and 1") may be modified such that the bonded region 34A is formed by a bonding means, such as heat sealing, high frequency sealing, or ultrasonic sealing, in place of or in addition to application of an adhesive.

The pull-on absorbent articles of the invention (e.g., the diapers 1A, 1B, and 1C) may be modified such that the rear longitudinally elasticized region Eb is formed only in the rear extension portion 21b and/or the front longitudinally elasticized region Ea is formed only in the front extension portion 21a; or that the rear and front longitudinally elasticized regions Eb and Ea are short of the respective longitudinal edges of the absorbent assembly.

The front panel 2A may dispense with the front longitudinally elasticized region Ea and/or the front extension portion 21a. For example, in the fourth to sixth embodiments, the elastic members 36 may be disposed only in the rear panel 2B and the crotch portion C; and in the seventh embodiment the elastic members 39 may be disposed only in the rear panel 2B and the crotch portion C. The number of the elastic members 36 disposed in each side sheet portion 34 may be changed from two to one or three or even more (e.g., three to five). The number of the elastic members 39 disposed in each standing gather-forming portion 38 may be one as in the sixth embodiment or three or more (e.g., three to five).

The pull-on absorbent articles of the invention are not limited to disposable pull-on diapers for children or adults but include pant-type sanitary napkins.

In the description given above, particulars of a certain embodiment that have been omitted to avoid redundancy can appropriately be complemented by the corresponding description of other embodiments. Particulars described as being characteristic of a certain embodiment can apply to other embodiments appropriately. Particulars of every embodiment are appropriately interchangeable between embodiments. For instance, the configuration of the front portion A used in any one of the fourth to seventh embodiments and the configuration of the rear portion B used in any one of the fourth to seventh embodiments may be combined.

### Industrial Applicability

The pull-on absorbent article of the invention is of the type wherein the outer cover is separated into a front panel adapted to be worn about the front of a wearer and a rear panel adapted to be worn about the rear of the wearer and yet provides an improved appearance from the back and side of the wearer.

The pull-on absorbent article of the invention is of the type wherein the outer cover is separated into a front panel adapted to be worn about the front of a wearer and a rear panel adapted to be worn about the rear of the wearer and yet provides good coverage over the wearer's buttocks and an improved appearance from the back and side of the wearer.

## Claims

1. A pull-on absorbent article (1, 1', 1", 1A, 1B, 1C, 1D) comprising a front panel (2A) adapted to be worn about the front of a wearer, a rear panel (2B) adapted to be worn about the rear of the wearer, and an absorbent assembly (3) fixed to the front and the rear panel (2A, 2B) so as to bridge them, the article (1, 1', 1", 1A, 1B, 1C, 1D) having a longitudinal direction and a lateral direction, and the article (1, 1', 1", 1A, 1B, 1C, 1D) having a pair of side seals (4) formed by joining the front panel (2A) and the rear panel (2B) along their lateral side edges (2a, 2b),
the rear panel (2B) having an oblong rectangular shape, being longer than the side seals (4) in the longitudinal direction (X) of the absorbent article (1, 1', 1", 1A, 1B, 1C, 1D) and having a rear extension portion (21b) extending downward from the side seals (4),
the absorbent assembly (3) comprising an absorbent member (33) oblong in the longitudinal direction (X) of the article (1, 1', 1 ", 1A, 1B, 1C, 1D) and a side sheet portion (34) located laterally outwardly from each lateral side edge of the absorbent member (33), each side sheet portion (34) having an elastic member (36) to form a side gather (34c) in at least the crotch portion (C) of the absorbent article (1, 1', 1", 1A, 1B, 1C, 1D),
each side sheet portion (34) having a non-bonded region (34N) not bonded to the rear extension portion (21b) in the portion thereof overlapping the rear panel (2B) and nearer the crotch portion (C),
the elastic member (36) extending in the non-bonded region (34N) so that the side sheet portion (34) is elasticized in the non-bonded region (34N),
the front panel (2A) having an oblong rectangular shape longer than the side seals (4) in the longitudinal direction (X) of the absorbent article (1, 1', 1", 1A, 1B, 1C, 1D) and having a front extension portion (21a) extending downward from the side seals (4),
wherein each side sheet portion (34) has a bonded region (34A) bonded to the rear panel (2B) in a portion thereof overlapping the rear panel ("B) and nearer the longitudinal end of the absorbent assembly (3).

2. The pull-on absorbent article according to claim 1, wherein the front extension portion (21a) has an elasticized extension region (G3) where an elastic member (24) is fixed so that the elasticized extension region (G3) is elasticized in a lateral direction of the absorbent article (1, 1',1", 1A, 1B, 1C, 1D).

3. The pull-on absorbent article according to claim 1 or 2, wherein the elastic member (36) forming each side gather (34c) in the crotch portion (C) comprises a plurality of elastic members, and all the elastic members are disposed closer to the free edge (34a) than the lateral center of the side gather (34c).

4. The pull-on absorbent article according to any one of claims 1 to 3, wherein the elastic member (36) forming each side gather (34c) is absent just along the free edge (34a) of the side gather (34c).

5. The pull-on absorbent article according to any one of claims 1 to 4, wherein the absorbent assembly (3) has its lateral middle portion, where the absorbent member (33) is disposed, bonded to a main portion of the rear panel (2B) located between the opposite side seals (4) and a main portion of the front panel (2A) located between the opposite side seals (4).

6. The pull-on absorbent article according to any one of claims 1 to 5, wherein the non-bonded region (34N) is not bonded to the rear panel (2B) in at least a part thereof laterally outward from the position of the elastic member (36).

7. The pull-on absorbent article according to any one of claims 1 to 6, wherein each side sheet portion (34) is bonded in its laterally outwardly lying position to the front panel (2A), and
each side sheet portion (34) is also elasticized in its part overlapping the front panel (2A).

8. The pull-on absorbent article according to any one of claims 1 to 7, wherein each side sheet portion (34) has an elasticized region (134c) with an elastic member fixed thereto in a stretched state, the elasticized region (134c) extending in the crotch portion (C) of the article (1, 1', 1", 1A, 1B, 1C, 1D) to be worn about the crotch of the wearer and on the rear extension portion (21 a, 21 b), and
each elasticized region (134c) is bonded, over at least a part of its width, to at least the rear extension portion (21b) of the rear panel (2B) to form a rear longitudinally elasticized region (Eb) extensible and contractible together with the elasticized region (134c) such that, in a relaxed state of the absorbent article (1, 1', 1", 1A, 1B, 1C, 1D) with each rear longitudinally elasticized region (Eb) contracted, the lower edge of the rear panel (2B) forms a downward concave near each rear longitudinally elasticized region (Eb).

9. The pull-on absorbent article according to claim 8, wherein the rear longitudinally elasticized region (Eb) is formed by bonding the elasticized region (134c) to the rear panel (2B) over a part of its width where the elastic member is disposed.

10. The pull-on absorbent article according to claim 8, wherein the rear longitudinally elasticized region (Eb) is formed by bonding the elasticized region (134c) to the rear panel (2B) over only a part of its width where the elastic member is absent.

11. The pull-on absorbent article according to any one of claims 8 to 10, wherein each elasticized region (134c) is bonded, over at least a part of its width, to the front panel (2A) to form a front longitudinally elasticized region (Ea) extensible and contractible together with the elasticized region (134c) such that, in a relaxed state of the absorbent article (1, 1', 1", 1A, 1B, 1C, 1D) with each front longitudinally elasticized region (Ea) contracted, the lower edge of the front panel (2A) forms a downward concave near each front longitudinally elasticized region (Ea).

12. The pull-on absorbent article according to any one of claims 8 to 11, wherein the absorbent assembly (3) is configured to form a standing gather laterally inwardly from each side sheet portion (34).

13. The pull-on absorbent article according to any one of claims 1 to 8, wherein the absorbent assembly (3) comprises a standing gather-forming portion (38) forming a standing gather (38c) laterally inward from each side sheet portion (34),
each side sheet portion (34) has an elasticized region (134c) with an elastic member fixed thereto in a stretched state, the elasticized region (134c) extending in the crotch portion (C) of the article to be worn about the crotch of the wearer and on the rear extension portion,
the elasticized region (134c) is non-bonded, over a part of its width where the elastic member is fixed, to the rear extension portion (21b),
the standing gather-forming portion (38) is configured to form the standing gather (38c) in the crotch portion (C) and on the rear extension portion (21b), and
the rear panel (2B) has, in at least the rear extension portion (21b) thereof, a rear longitudinally elasticized region (Eb) extensible and contractible in the longitudinal direction of the absorbent assembly (3) near each lateral side edge of the absorbent member (33) such that, in a relaxed state of the absorbent article (1, 1', 1", 1A, 1B, 1C, 1D) with each rear longitudinally elasticized region (Eb) contracted, the lower edge of the rear panel (2B) forms a downward concave near each rear longitudinally elasticized region (Eb).

14. The pull-on absorbent article according to claim 13, wherein the absorbent member (33) has a central core segment, a pair of side core segment, and a pair of flexing regions (3J) located in the crotch portion (C) between the central core segment and the respective side core segments, each flexing region (3J) being a cutout region where material making the central and side core segments is missing or a low weight region having a lower basis weight of the material than other regions.

15. The pull-on absorbent article according to any one of claims 8 to 14, wherein each rear longitudinally elasticized region (Eb) extends over a length straddling the rear extension portion (21b) and a main portion of the rear panel (2B) located between the opposite side seals (4).

16. The pull-on absorbent article according to claim 11, wherein the front longitudinally elasticized region (Ea) extends over a length straddling the front extension portion (21a) and a main portion of the front panel (2A) located between the opposite side seals (4).

## Patentansprüche

1. Absorbierender Artikel des Höschentyps (1, 1', 1", 1A, 1B, 1C, 1D) aufweisend einen vorderen Streifen (2A), der beim Tragen um die Vorderseite eines Trägers angeordnet ist, einen hinteren Streifen (2B), der beim Tragen um die Rückseite eines Trägers angeordnet ist, und eine absorbierende Anordnung (3), die an dem vorderen und dem hinteren Streifen (2A, 2B) angebracht ist, um sie zu überbrücken, wobei der Artikel (1, 1', 1", 1A, 1B, 1C, 1D) eine Längsrichtung und eine laterale Richtung hat und der Artikel (1, 1', 1'', 1 A, 1B, 1C, 1 D) ein Paar Seitenversiegelungen (4) aufweist, die durch Verbinden des vorderen Streifens (2A) mit dem hinteren Streifen (2B) entlang ihrer lateralen Seitenkanten (2a, 2b) gebildet sind,
wobei der hintere Streifen (2B) eine längliche rechteckige Form hat, die in der Längsrichtung (X) des absorbierenden Artikels (1, 1', 1", 1A, 1 B, 1C, 1 D) länger als die Seitenversiegelungen (4) ist und einen hinteren Verlängerungsabschnitt (21b) hat, der sich von den Seitenversiegelungen (4) abwärts erstreckt,
wobei die absorbierende Anordnung (3) ein in der Längsrichtung (X) des Artikels (1, 1', 1", 1A, 1B, 1C, 1D) längliches absorbierendes Element (33) und einen Seitenschichtabschnitt (34) aufweist, der lateral auswärts von jeder lateralen Seitenkante des absorbierenden Elements (33) angeordnet ist, wobei jeder Seitenschichtabschnitt (34) ein elastisches Element (36) aufweist, um eine Seitenkräuselung (34c) zumindest in dem Schrittabschnitt (C) des absorbierenden Artikels (1, 1', 1", 1A, 1B, 1C, 1D) zu bilden,
wobei jeder Seitenschichtabschnitt (34) in dem Teil, der den hinteren Streifen (2B) überlappt und näher am Schrittabschnitt (C) ist, einen nichtgebundenen Bereich (34N) aufweist, der nicht mit dem hinteren Verlängerungsabschnitt (21b) verbunden ist,
das elastische Element (36) sich in dem nichtgebundenen Bereich (34N) erstreckt, so dass der Seitenschichtabschnitt (34) in dem nichtgebundenen Bereich (34N) elastisch gemacht ist,
der vordere Streifen (2A) eine längliche rechteckige Form hat, die in der Längsrichtung (X) des absorbierenden Artikels (1, 1', 1", 1A, 1B, 1C, 1D) länger als die Seitenversiegelungen (4) ist und einen vorderen Verlängerungsabschnitt (21a) hat, der sich von den Seitenversiegelungen (4) abwärts erstreckt,
wobei jeder Seitenschichtabschnitt (34) in dem Teil, der den hinteren Streifen (2B) überlappt und näher am Längsende der absorbierenden Anordnung (3) ist, einen gebundenen Bereich (34A) aufweist, der mit dem hinteren Streifen (2B) verbunden ist.

2. Absorbierender Artikel des Höschentyps nach Anspruch 1, wobei der vordere Verlängerungsabschnitt (21a) einen elastisch gemachten Verlängerungsbereich (G3) hat, wo ein elastisches Element (24) so angebracht ist, dass der elastisch gemachte Bereich (G3) in einer lateralen Richtung des absorbierenden Artikels (1, 1', 1", 1A, 1B, 1C, 1D) elastisch gemacht ist.

3. Absorbierender Artikel des Höschentyps nach Anspruch 1 oder 2, wobei das elastische Element (36), das jede Seitenkräuselung (34c) in dem Schrittabschnitt (C) bildet, mehrere elastische Elemente aufweist, und alle elastischen Elemente näher an der freien Kante (34a) angeordnet sind als das laterale Zentrum der Seitenkräuselung (34c).

4. Absorbierender Artikel des Höschentyps nach einem der Ansprüche 1 bis 3, wobei das elastische Element (36), das jede Seitenkräuselung (34c) bildet, genau entlang der freien Kante (34a) der Seitenkräuselung (34c) nicht vorhanden ist.

5. Absorbierender Artikel des Höschentyps nach einem der Ansprüche 1 bis 4, wobei die absorbierende Anordnung (3) ihren lateralen Mittelabschnitt, wo das absorbierende Element (33) angeordnet ist, mit einem zwischen den gegenüberliegenden Seitenversiegelungen (4) angeordneten Hauptabschnitt des hinteren Streifens (2B) und mit einem zwischen den gegenüberliegenden Seitenversiegelungen (4) angeordneten Hauptabschnitt des vorderen Streifens (2A) verbunden hat.

6. Absorbierender Artikel des Höschentyps nach einem der Ansprüche 1 bis 5, wobei der nichtgebundene Bereich (34N) zumindest in seinem Teil lateral auswärts von der Position des elastischen Elements (36) nicht mit dem hinteren Streifen (2B) verbunden ist.

7. Absorbierender Artikel des Höschentyps nach einem der Ansprüche 1 bis 6, wobei jeder Seitenschichtabschnitt (34) in seiner lateral auswärts liegenden Position mit dem vorderen Streifen (2A) verbunden ist und jeder Seitenschichtabschnitt (34) in seinem den vorderen Streifen (2A) überlappenden Teil auch elastisch gemacht ist.

8. Absorbierender Artikel des Höschentyps nach einem der Ansprüche 1 bis 7, wobei jeder Seitenschichtabschnitt (34) einen elastisch gemachten Bereich (134c) mit einem elastischen Element aufweist, das in einem gedehnten Zustand daran angebracht ist, wobei sich der elastisch gemachte Bereich (134c) in dem beim Tragen um den Schritt des Trägers angeordneten Schrittabschnitt (C) des Artikels (1, 1', 1 ", 1A, 1B, 1C, 1D) und an dem hinteren Verlängerungsabschnitt (21a, 21b) erstreckt, und
jeder elastisch gemachte Bereich (134c) zumindest über einen Teil seiner Breite zumindest mit dem hinteren Verlängerungsabschnitt (21b) des hinteren Streifens (2B) verbunden ist, um einen hinteren in Längsrichtung elastisch gemachten Bereich (Eb) zu bilden, der zusammen mit dem elastisch gemachten Bereich (134c) dehnbar und zusammenziehbar ist, so dass in einem entspannten Zustand des absorbierenden Artikels (1, 1', 1", 1A, 1B, 1C, 1D), in welchem jeder hintere in Längsrichtung elastisch gemachte Bereich (Eb) zusammengezogen ist, die untere Kante des hinteren Streifens (2B) in der Nähe jedes hinteren in Längsrichtung elastisch gemachten Bereichs (Eb) eine Abwärtswölbung bildet.

9. Absorbierender Artikel des Höschentyps nach Anspruch 8, wobei der hintere in Längsrichtung elastisch gemachte Bereich (Eb) dadurch gebildet ist, dass der elastisch gemachte Bereich (134c) über einen Teil seiner Breite, wo das elastische Element angeordnet ist, mit dem hinteren Streifen (2B) verbunden ist.

10. Absorbierender Artikel des Höschentyps nach Anspruch 8, wobei der hintere in Längsrichtung elastisch gemachte Bereich (Eb) dadurch gebildet ist, dass der elastisch gemachte Bereich (134c) nur über einen Teil seiner Breite, wo das elastische Element nicht vorhanden ist, mit dem hinteren Streifen (2B) verbunden ist.

11. Absorbierender Artikel des Höschentyps nach einem der Ansprüche 8 bis 10, wobei jeder elastisch gemachte Bereich (134c) zumindest über einen Teil seiner Breite mit dem vorderen Streifen (2A) verbunden ist, um einen vorderen in Längsrichtung elastisch gemachten Bereich (Ea) zu bilden, der zusammen mit dem elastisch gemachten Bereich (134c) dehnbar und zusammenziehbar ist, so dass in einem entspannten Zustand des absorbierenden Artikels (1, 1', 1'', 1A, 1B, 1C, 1D), in welchem jeder vordere in Längsrichtung elastisch gemachte Bereich (Ea) zusammengezogen ist, die untere Kante des vorderen Streifens (2A) in der Nähe jedes vorderen in Längsrichtung elastisch gemachten Bereichs (Ea) eine Abwärtswölbung bildet.

12. Absorbierender Artikel des Höschentyps nach einem der Ansprüche 8 bis 11, wobei die absorbierende Anordnung (3) konfiguriert ist, lateral einwärts von jedem Seitenschichtabschnitt (34) eine Stehkräuselung zu bilden.

13. Absorbierender Artikel des Höschentyps nach einem der Ansprüche 1 bis 8, wobei die absorbierende Anordnung (3) einen Stehkräuselung-Bildungsabschnitt (38) aufweist, der lateral einwärts von jedem Seitenschichtabschnitt (34) eine Stehkräuselung (38c) bildet,
wobei jeder Seitenschichtabschnitt (34) einen elastisch gemachten Bereich (134c) mit einem elastischen Element aufweist, das in einem gedehnten Zustand daran angebracht ist, wobei sich der elastisch gemachte Bereich (134c) in dem beim Tragen um den Schritt des Trägers angeordneten Schrittabschnitt (C) des Artikels und an dem hinteren Verlängerungsabschnitt erstreckt, und
der elastisch gemachte Bereich (134c) über einen Teil seiner Breite, wo das elastische Element angebracht ist, nicht mit dem hinteren Verlängerungsabschnitt (21b) verbunden ist,
der Stehkräuselung-Bildungsabschnitt (38) konfiguriert ist, in dem Schrittabschnitt (C) und an dem hinteren Verlängerungsabschnitt (21b) die Stehkräuselung (38c) zu bilden, und
der hintere Streifen (2B) zumindest in seinem hinteren Verlängerungsabschnitt (21b) einen hinteren in Längsrichtung elastisch gemachten Bereich (Eb) aufweist, der in der Nähe jeder lateralen Seitenkante des absorbierenden Elements (33) in Längsrichtung der absorbierenden Anordnung (3) dehnbar und zusammenziehbar ist, so dass in einem entspannten Zustand des absorbierenden Artikels (1, 1', 1", 1A, 1B, 1C, 1D), in welchem jeder hintere in Längsrichtung elastisch gemachte Bereich (Eb) zusammengezogen ist, die untere Kante des hinteren Streifens (2B) in der Nähe jedes hinteren in Längsrichtung elastisch gemachten Bereichs (Eb) eine Abwärtswölbung bildet.

14. Absorbierender Artikel des Höschentyps nach Anspruch 13, wobei das absorbierende Element (33) ein zentrales Kernsegment, ein Paar Seitenkernsegmente und ein Paar Biegebereiche (3J) aufweist, die in dem Schrittabschnitt (C) zwischen dem zentralen Kernsegment und den jeweiligen Seitenkernsegmenten angeordnet sind, wobei jeder Biegebereich (3J) ein ausgeschnittener Bereich, wo Material, das das zentrale Kernsegment und die Seitenkernsegmente bildet, fehlt, oder ein Niedriggewichtbereich mit einem niedrigeren Basisgewicht des Materials als andere Bereiche ist.

15. Absorbierender Artikel des Höschentyps nach einem der Ansprüche 8 bis 14, wobei jeder hintere in Längsrichtung elastisch gemachte Bereich (Eb) sich über eine Länge erstreckt, die den hinteren Verlängerungsabschnitt (21b) und einen zwischen den gegenüberliegenden Seitenversiegelungen (4) angeordneten Hauptabschnitt des hinteren Streifens (2B) überspannt.

16. Absorbierender Artikel des Höschentyps nach Anspruch 11, wobei der vordere in Längsrichtung elastisch gemachte Bereich (Ea) sich über eine Länge erstreckt, die den vorderen Verlängerungsabschnitt (21 a) und einen zwischen den gegenüberliegenden Seitenversiegelungen (4) angeordneten Hauptabschnitt des vorderen Streifens (2A) überspannt.

## Revendications

1. Article absorbant à enfiler (1, 1', 1'', 1A, 1B, 1C, 1D) comprenant un panneau avant (2A) adapté pour être porté autour de la partie avant d'un utilisateur, un panneau arrière (2B) adapté pour être porté autour de la partie arrière de l'utilisateur, et un ensemble absorbant (3) fixé sur le panneau avant et le panneau arrière (2A, 2B) afin de les relier, l'article (1, 1', 1'', 1A, 1B, 1C, 1D) ayant une direction longitudinale et une direction latérale, et l'article (1, 1', 1'', 1A, 1B, 1C, 1D) ayant une paire de joints latéraux.(4) formés en assemblant le panneau avant (2A) et le panneau arrière (2B) le long de leurs bords latéraux (2a, 2b),
le panneau arrière (2B) ayant une forme rectangulaire oblongue, qui est plus longue que les joints latéraux (4) dans la direction longitudinale (X) de l'article absorbant (1, 1', 1", 1A, 1B, 1C, 1D) et ayant une partie d'extension arrière (21b) s'étendant vers le bas à partir des joints latéraux (4),
l'ensemble absorbant (3) comprenant un élément absorbant (33) oblong dans la direction longitudinale (X) de l'article (1, 1', 1'', 1A, 1B, 1C, 1D) et une partie de feuille latérale (34) positionnée latéralement vers l'extérieur à partir de chaque bord latéral de l'élément absorbant (33), chaque partie de feuille latérale (34) ayant un élément élastique (36) pour former une fronce latérale (34c) dans au moins la partie d'entrejambes (C) de l'article absorbant (1, 1', 1", 1A, 1B, 1C, 1D),
chaque partie de feuille latérale (34) ayant une région non reliée (34N) non reliée à la partie d'extension arrière (21b) dans sa partie chevauchant le panneau arrière (2B) et plus à proximité de la partie d'entrejambes (C),
l'élément élastique (36) s'étendant dans la partie non reliée (34N) de sorte que la partie de feuille latérale (34) est élastiquée dans la région non reliée (34N),
le panneau avant (2A) ayant une forme rectangulaire oblongue plus longue que les joints latéraux (4) dans la direction longitudinale (X) de l'article absorbant (1, 1', 1'', 1A, 1B, 1C, 1D) et ayant une partie d'extension avant (21a) s'étendant vers le bas à partir des joints latéraux (4),
dans lequel chaque partie de feuille latérale (34) a une région reliée (34A) reliée au panneau arrière (2B) dans sa partie chevauchant le panneau arrière (2B) et plus à proximité de l'extrémité longitudinale de l'ensemble absorbant (3).

2. Article absorbant à enfiler selon la revendication 1, dans lequel la partie d'extension avant (21a) a une région d'extension élastiquée (G3) dans laquelle un élément élastique (24) est fixé de sorte que la région d'extension élastiquée (G3) est élastiquée dans une direction latérale de l'article absorbant (1, 1', 1'', 1A, 1B, 1C, 1D).

3. Article absorbant à enfiler selon la revendication 1 ou 2, dans lequel l'élément élastique (36) formant chaque fronce latérale (34c) dans la partie d'entrejambes (C) comprend une pluralité d'éléments élastiques et tous les éléments élastiques sont disposés plus à proximité du bord libre (34a) que le centre latéral de la fronce latérale (34c).

4. Article absorbant à enfiler selon l'une quelconque des revendications 1 à 3, dans lequel l'élément élastique (36) formant chaque fronce latérale (34c) n'est absent que le long du bord libre (34a) de la fronce latérale (34c).

5. Article absorbant à enfiler selon l'une quelconque des revendications 1 à 4, dans lequel l'ensemble absorbant (3) a sa partie centrale latérale, où l'élément absorbant (33) est disposé, relié à une partie principale du panneau arrière (2B) positionnée entre les joints latéraux (4) opposés et une partie principale du panneau avant (2A) positionnée entre les joints latéraux (4) opposés.

6. Article absorbant à enfiler selon l'une quelconque des revendications 1 à 5, dans lequel la région non reliée (34N) n'est pas reliée au panneau arrière (2B) au moins dans sa partie latéralement vers l'extérieur de la position de l'élément élastique (36).

7. Article absorbant à enfiler selon l'une quelconque des revendications 1 à 6, dans lequel chaque partie de feuille latérale (34) est reliée, dans sa position allongée latéralement vers l'extérieur, au panneau avant (2A), et
chaque partie de feuille latérale (34) est également élastiquée dans sa partie chevauchant le panneau avant (2A).

8. Article absorbant à enfiler selon l'une quelconque des revendications 1 à 7, dans lequel chaque partie de feuille latérale (34) a une région élastiquée (134c) avec un élément élastique fixé à cette dernière dans un état étiré, la région élastiquée (134c) s'étendant dans la partie d'entrejambes (C) de l'article (1, 1', 1", 1A, 1B, 1C, 1D) à porter autour de l'entrejambes de l'utilisateur et sur la partie d'extension arrière (21a, 21b), et
chaque région élastiquée (134c) est reliée, sur au moins une partie de sa largeur, au moins à la partie d'extension arrière (21b) du panneau arrière (2B) pour former une région longitudinalement élastiquée arrière (Eb) extensible et contractile conjointement avec la région élastiquée (134c) de sorte que, à l'état détendu de l'article absorbant (1, 1', 1", 1A, 1B, 1C, 1D) avec chaque région longitudinalement élastiquée arrière (Eb) contractée, le bord inférieur du panneau arrière (2B) forme une concave vers le bas à proximité de chaque région longitudinalement élastiquée arrière (Eb).

9. Article absorbant à enfiler selon la revendication 8, dans lequel la région longitudinalement élastiquée arrière (Eb) est formée en reliant la région élastiquée (134c) au panneau arrière (2B) sur une partie de sa largeur où l'élément élastique est disposé.

10. Article absorbant à enfiler selon la revendication 8, dans lequel la région longitudinalement élastiquée arrière (Eb) est formée en reliant la région élastiquée (134c) au panneau arrière (2B) sur une seule partie de sa largeur où l'élément élastique est absent.

11. Article absorbant à enfiler selon l'une quelconque des revendications 8 à 10, dans lequel chaque région élastiquée (134c) est reliée, sur au moins une partie de sa largeur, au panneau avant (2A) afin de former une région longitudinalement élastiquée avant (Ea) extensible et contractile conjointement avec la région élastiquée (134c) de sorte que, dans un état détendu de l'article absorbant (1, 1', 1", 1A, 1B, 1C, 1D) avec chaque région longitudinalement élastiquée avant (Ea) contractée, le bord inférieur du panneau avant (2A) forme une concave vers le bas à proximité de la région longitudinalement élastiquée avant (Ea).

12. Article absorbant à enfiler selon l'une quelconque des revendications 8 à 11, dans lequel l'ensemble absorbant (3) est configuré pour former une fronce fixe latéralement vers l'avant à partir de chaque partie de feuille latérale (34).

13. Article absorbant à enfiler selon l'une quelconque des revendications 1 à 8, dans lequel l'ensemble absorbant (3) comprend une partie de formation de fronce fixe (38) formant une fronce fixe (38c) latéralement vers l'intérieur à partir de chaque partie de feuille latérale (34),
chaque partie de feuille latérale (34) a une région élastiquée (134c) avec un élément élastique fixé à cette dernière dans un état étiré, la région élastiquée (134c) s'étendant dans la partie d'entrejambes (C) de l'article à porter autour de l'entrejambes de l'utilisateur et sur la partie d'extension arrière,
la région élastiquée (134c) est non reliée, sur une partie de sa largeur où l'élément élastique est fixé, à la partie d'extension arrière (21b),
la partie de formation de fronce fixe (38) est configurée pour former la fronce fixe (38c) dans la partie d'entrejambes (C) et sur la partie d'extension arrière (21b), et
le panneau arrière (2B) a, dans au moins sa partie d'extension arrière (21b), une région longitudinalement élastiquée arrière (Eb) extensible et contractile dans la direction longitudinale de l'ensemble absorbant (3) à proximité de chaque bord latéral de l'élément absorbant (33) de sorte que, dans un état détendu de l'article absorbant (1, 1', 1'', 1A, 1B, 1C, 1D) avec chaque région longitudinalement élastiquée arrière (Eb) contractée, le bord inférieur du panneau arrière (2B) forme une concave vers le bas à proximité de chaque région longitudinalement élastiquée arrière (Eb).

14. Article absorbant à enfiler selon la revendication 13, dans lequel l'élément absorbant (33) a un segment d'âme central, une paire de segments d'âme latéraux, et une paire de régions de flexion (3J) positionnées dans la partie d'entrejambes (C) entre le segment d'âme central et les segments d'âme latéraux respectifs, chaque région de flexion (3J) étant une région découpée où le matériau réalisant les segments d'âme central et latéraux manque ou une région de poids inférieur ayant un poids de base inférieur de matériau par rapport aux autres régions.

15. Article absorbant à enfiler selon l'une quelconque des revendications 8 à 14, dans lequel chaque région longitudinalement élastiquée arrière (Eb) s'étend sur une longueur à cheval sur la partie d'extension arrière (21b) et une partie principale du panneau arrière (2B) positionnée entre les joints latéraux (4) opposés.

16. Article absorbant à enfiler selon la revendication 11, dans lequel la région longitudinalement élastiquée avant (Ea) s'étend sur une longueur à cheval sur la partie d'extension avant (21a) et une partie principale du panneau avant (2A) positionnée entre les joints latéraux (4) opposés.
